# FASCICULE DE BREVET EUROPEEN

(11) **EP 0 812 359 B1**
(45) Date de publication et mention de la délivrance du brevet: **09.11.2005**
(21) Numéro de dépôt: 96904904.8
(22) Date de dépôt: 26.02.1996
(51) Int. Cl.: C12N 15/49, C12N 7/00, C12Q 1/70, C07K 14/16, A61K 39/21, G01N 33/569, C07K 16/10

(54) **VIH-1 DE GROUPE O, FRAGMENTS DESDITS VIRUS, AINSI QUE LEURS APPLICATIONS**
HIV-1 DER GRUPPE 0, ENTSPRECHENDE FRAGMENTE DIESES VIRUS,SOWIE DEREN ANWENDUNGEN
GROUP O HIV-1, FRAGMENTS OF SUCH VIRUSES, AND USES THEREOF

(30) Priorité: 27.02.1995 FR 9502236
(43) Date de publication de la demande: 17.12.1997
(73) Titulaire: INSTITUT NATIONAL DE LA SANTE ET DE LA RECHERCHE MEDICALE (INSERM), 75654 Paris Cédex 13 (FR); ASSISTANCE PUBLIQUE, HOPITAUX DE PARIS, 75100 Paris (FR)
(72) Inventeur: SIMON, François, F-75018 Paris (FR); SARAGOSTI, Sentob, F-92100 Boulogne-Billancourt (FR); LOUSSERT-AJAKA, Ibtissam, F-78500 Sartrouville (FR); LY, Thoai-Duong, F-92500 Rueil-Malmaison (FR); CHAIX-BAUDIER, Marie-Laure, F-75011 Paris (FR)
(74) Mandataire: Orès, Irène
(86) Numéro de dépôt international: PCT/FR1996/000294
(87) Numéro de publication internationale: WO 1996/027013

(56) Documents cités:
- LANCET THE, vol. 343, 4 Juin 1994, LONDON GB, pages 1393-1394, XP002005313 I.LOUSSERT-AJAKA ET AL.: "HIV-1/HIV-2 seronegativity in HIV-1 subtype O infected patients" cité dans la demande
- VIROLOGY, vol. 205, no. 1, 15 Novembre 1994, ORLANDO US, pages 247-253, XP002005314 P.CHARNEAU ET AL.: "Isolation and envelope sequence of a highly divergent HIV-1 isolate: Definition of a new HIV-1 group"
- AIDS(US), vol. 8, no. 8, pages 1405-1412, XP002005315 J.N.NKENGASONG ET AL.: "Genomic subtypes of HIV-1 in Cameroon"

### LISTE DE SEQUENCES

(1) INFORMATIONS GENERALES:
   (i) DEPOSANT:
      (A) NOM: INSTITUT NATIONAL DE LA RECHERCHE MEDICALE -INSERM
      (B) RUE: 101 RUE DE TOLBIAC
      (C) VILLE: PARIS
      (E) PAYS: FRANCE
      (F) CODE POSTAL: 75654 CEDEX 13

      (A) NOM: ASSISTANCE PUBLIQUE - HOPITAUX DE PARIS
      (B) RUE: 3 AVENUE VICTORIA
      (C) VILLE: PARIS
      (E) PAYS: FRANCE
      (F) CODE POSTAL: 75004

      (A) NOM: SIMON Francois
      (B) RUE: 8 rue Germain Pilon
      (C) VILLE: PARIS
      (E) PAYS: FRANCE
      (F) CODE POSTAL: 75018

      (A) NOM: SARAGOSTI Sentob
      (B) RUE: 69 bis rue de Billancourt
      (C) VILLE: BOULOGNE-BILLANCOURT
      (E) PAYS: FRANCE
      (F) CODE POSTAL: 92100

      (A) NOM: LOUSSERT-AJAKA Ibtissam
      (B) RUE: 26 avenue de la REPUBLIQUE
      (C) VILLE: SARTROUVILLE
      (E) PAYS: FRANCE
      (F) CODE POSTAL: 78500

      (A) NOM: LY Thoai-Duong
      (B) RUE: 22 rue PERREIRE
      (C) VILLE: RUEIL-MALMAISON
      (E) PAYS: FRANCE
      (F) CODE POSTAL: 92500

      (A) NOM: CHAIX-BAUDIER Marie-Laure
      (B) RUE: 37 rue Godefroy Cavaignac
      (C) VILLE: PARIS
      (E) PAYS: FRANCE
      (F) CODE POSTAL: 75011
   (ii) TITRE DE L' INVENTION: VIH-1 DE GROUPE O, FRAGMENTS DESDITS VIRUS ET LEURS APPLICATIONS.
   (iii) NOMBRE DE SEQUENCES: 77
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
   (vi) DONNEES DE LA DEMANDE ANTERIEURE:
      (A) NUMERO DE LA DEMANDE: FR 9502236
      (B) DATE DE DEPOT: 27-FEB-1995
(2) INFORMATIONS POUR LA SEQ ID NO: 1:
   (i) CARACTERISTIQUES DE LA SEQUENCE:
      (A) LONGUEUR: 291 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 1:
   GTGGTTACTT GTACACATGG CATCAAGCCA ACAGTAAGTA CTCAGCTAAT ATTAAATGGA  60
   ACACTCTCAG AAGGAAAGAT AAGAATGATG GCAAAAAATA TTTCGGATAG TGGCCAAAAT 120
   ATCATAGTGA CCCTAAATAC TACTATAAAC ATGACCTGCC AGAGACCAGG ACATCAAACA 180
   GTACAAGAGA TAAGGATAGG TCCAATGGCC TGGTACAGCA TGGGCTTAGC GGCAGGAAAC 240
   GGATCTGAGT CAAGAAGAGC TTATTGTGAA TATAATACCA CTAATTGGAT A          291
(2) INFORMATIONS POUR LA SEQ ID NO: 2:
   (i) CARACTERISTIQUES DE LA SEQUENCE:
      (A) LONGUEUR: 294 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 2:
   GTAGTTACTT GTACACATGG CATCAAGCCA ACAGTGAGTA CTCATCTAAT ATTAAATGGG  60
   ACAATCTCTG AAGGAGAAAT AAGAATTATG GGAAAAAATA TTCGGGAAAA TGCTAAAAAT 120
   ATCATAGTGA CCCTAAATTC TACTATAAAC ATGACCTGTG AGAGACCAGA GGGAAATCTG 180
   ACAATACAAG AGATACACTC AGGACCAATG GCCTGGTACA GCTTGGGACT AAAGAGAAAT 240
   ACAACCGTAA GATCAAGATC AGCTCATTGC AAGTATAACA CCACTAATTG GGAA       294
(2) INFORMATIONS POUR LA SEQ ID NO: 3:
   (i) CARACTERISTIQUES DE LA SEQUENCE:
      (A) LONGUEUR: 297 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 3:
   GTGGTTACTT GTACACATGG CATCAAGCCA GCAGTAAGTA CTCAGCTAAT ATTAAATGGG  60
   ACACTCTCTA AAGGAAAAAT AAGAATTATG GCAAAAAATA TTACAAACAC TGGGAATAAT 120
   ATCATAGTGA CTCTAAATTC CACCATAAAC ATAACCTGTA ACAGACCAGG AAGGGGAATA 180
   AAACAGATAG GTATAGGTCC AATGTCCGTA TACAGCGGGA GCTTAGCGGA CTTAGGGGGA 240
   AACAACAACT CAAGGATAGC TTATTGCGAT TATGACATCA CTAAGTGGAA CGAAACA    297
(2) INFORMATIONS POUR LA SEQ ID NO: 4:
   (i) CARACTERISTIQUES DE LA SEQUENCE:
      (A) LONGUEUR: 294 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 4:
   GTAGTTACTT GTACACATGG CATCAAGCCA ACAGTAAGTA CTCAATTAAT AATGAATGGG  60
   ACACTCTCTA GAGGGAAGAT AAGAATTATG GGAAGAAATA TTACAGACAA TACAAAGAAT 120
   ATTATAGTAA CCTTAAACAC TTCTATAAAC ATGACATGTA TGAGAAAAGG AAGAGGTAAA 180
   ATACAAAGGA TAGCGACAGG TCCACTGCGA TGGGTCAGTA TGGCAGCTAA AACAGAGTCA 240
   CAGAACACAG GGTCAAGGAT AGCTTATTGT ATGTATAATA ACACTGAATG GATA       294
(2) INFORMATIONS POUR LA SEQ ID NO: 5:
   (i) CARACTERISTIQUES DE LA SEQUENCE:
      (A) LONGUEUR: 291 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 5:
   GTGGTTACTT GTACACATGG CATCAAGCCA ACAGTAAGTA CTCAGCTAAT ATTAAATGGA  60
   ACACTCTCGA AAGGAAAGAT AAGACTGATG GCAAAAAATA TTTCGGATAG TGGCCAAAAT 120
   ATCATAGTGA CCCTAAATAC TACTATAAAC ATGACCTGCC ATAGACCAGG AAATCTAAAA 180
   GTACAGGAGA TAAAGATAGG TCCAATGGCC TGGTACAGCA TGGGCATAGA GAATGAAAAC 240
   ATACCTGATT CAAGAAAAGC TTATTGTGAT TATAATACCA CTAAGTGGGT A          291
(2) INFORMATIONS POUR LA SEQ ID NO: 6:
   (i) CARACTERISTIQUES DE LA SEQUENCE:
      (A) LONGUEUR: 291 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 6:
   GTAGTTACTT GTACACATGG CATCAAGCCC ACAGTGAGTA CTCAACTGAT ATTAAATGGG  60
   ACACTCTCTG AAAAGGGAAT AAGAATTATG GGAAAAAACA TTTCAAAAAC TGGGGAAAAT 120
   ATCATAGTGA CCCTAAATGT AAGCATAAAC ATTACTTGTC ATAGACCAGG AAATCTGTCA 180
   GTACAAGAGA TGAAAATAGG TCCACTGTCC TGGTACAGCA TGGGCCTAGC GGCAAACTCA 240
   AGCATAAAGT CAAGGGTAGC TTATTGCAAT TATAGTACCA CTGAATGGAC A          291
(2) INFORMATIONS POUR LA SEQ ID NO: 7:
   (i) CARACTERISTIQUES DE LA SEQUENCE:
      (A) LONGUEUR: 296 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 7:
   GTGGTTACTT GACACATGGC ATCAAGCCAG CAGTAAGTAC TCAACTAATA CTAAATGGGA  60
   CACTCTCTGA AGGGAAGATA AGAATTATGG GACAAAATAT CTCTGACAGT GGAAAGAATA 120
   TCATAGTAAC CCTAAATAAG ACTGTAAACA TGAACATAAC CTGCACAAGA GATGGAGATC 180
   AGAAGGTACA AGAGATAGGG ATAGGTCCAC TGTCATGGTA CAGTATGAGC ATTGCAGAAG 240
   ACAGCGCTAA AAACACAAGA GCAGCTTATT GTAACTATAG TGCAAGTAGT TGGAAG     296
(2) INFORMATIONS POUR LA SEQ ID NO: 8:
   (i) CARACTERISTIQUES DE LA SEQUENCE:
      (A) LONGUEUR: 120 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 8:
   AGACAACTCC GAGCTCGCCT GCTAGCCTTA GAAACCTTAA TACAGAATCA GCAACTCCTA  60
   AACTCGTGGG GCTGTAAGGG AAGGATAGTC TGCTACACAT CAGTAAAATG GAACTGGACA 120
(2) INFORMATIONS POUR LA SEQ ID NO: 9:
   (i) CARACTERISTIQUES DE LA SEQUENCE:
      (A) LONGUEUR: 120 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 9:
   AGACAACTCC GAGCTCGCCT GCTAGCCTTA GAAACCTTAA TACAGAATCA GCAACTCCTA  60
   AACCTATGGG GCTGTAAGGG AAGGCTACTC TGCTACACAT CAGTAAAATG GAATACGACA 120
(2) INFORMATIONS POUR LA SEQ ID NO: 10:
   (i) CARACTERISTIQUES DE LA SEQUENCE:
      (A) LONGUEUR: 120 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 10:
   AGACAACTCC GAGCTCGCCT GCAAGCCTTA GAAACCTTAA TCCAGAATCA GCAACTCCTA  60
   AGCCTGTGGG GCTGTAAAGG AAGGCTAGTC TGCTACACAT CAGTAAAATG GCACAACACA 120
(2) INFORMATIONS POUR LA SEQ ID NO: 11:
   (i) CARACTERISTIQUES DE LA SEQUENCE:
      (A) LONGUEUR: 120 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 11:
   AGACAACTCC GAGCTCGCCT GCAAGCCTTA GAACCCCTTA TACAGAATCA GCAACGCCTA 60
   AGCCTATGGG GATGTAAGGG AAGGATAATA TGTTACACAT CAGCAAAATG GAACAACACA 120
(2) INFORMATIONS POUR LA SEQ ID NO: 12:
   (i) CARACTERISTIQUES DE LA SEQUENCE:
      (A) LONGUEUR: 120 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 12:
   AGACAACTCC GAGCTCGCCT GCTAGCCTTA GAAACCTTAA TACAGAATCA GCAACTCCTA 60
   AACTCATGGG GCTGTAAGGG AAGGCTAGTC TGTTACACAT CAGTAAAATG GAACGAGACA 120
(2) INFORMATIONS POUR LA SEQ ID NO: 13:
   (i) CARACTERISTIQUES DE LA SEQUENCE:
      (A) LONGUEUR: 120 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 13:
   AGACAACTCC GAGCTCGCCT GCTAGCCTTA GAAACCTTGA TACAGAATCA GCAACTCCTA 60
   AACCTATGGG GCTGTAAGGG AAGGCTACTC TGCTACACAT CAGTAAAATG GAACAGTACA 120
(2) INFORMATIONS POUR LA SEQ ID NO: 14:
   (i) CARACTERISTIQUES DE LA SEQUENCE:
      (A) LONGUEUR: 120 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 14:
   AGACAACTCC GAGCTCGCCT GGTTGCCTTA GAAACCCTTG TACAGAATCA GCAACTCCTA 60
   AACCTATGGG GCTGTAAAGG AAGACTAACA TGCTATACAT CAGTAAAATG GAATGACACA 120
(2) INFORMATIONS POUR LA SEQ ID NO: 15:
   (i) CARACTERISTIQUES DE LA SEQUENCE:
      (A) LONGUEUR: 399 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 15:
   CCCATTTCTC CTAGAACTTT AAATGCATGG GTAAAGGCAG TAGAAGAGAA AGCCTTTAAC  60
   CCTGAAATCA TTCCTATGTT CATGGCATTG TCAGAGGGAG CTGTTCCCTA TGATATTAAT 120
   ACTATGCTAA ATGCCATAGG AGAACATCAA GGGGCTTTAC AAGTGCTAAA GGAAGTAATC 180
   AATGAGGAAG CATTGGAGTG GGATAGAACT CACCCACCAC CGATAGGGCC GTTACCACCA 240
   GGGCAGATAA GGGACCCAAC AGGAAGTGAC ATTGCTGGAA CAACTAGCAC TCAGCAAGAG 300
   CAAGTTCACT GGGTGACCAG GAACCCCAAC CCTATCCCAG TAGGAGACAT CTATTGGAAA 360
   TGGATAGTGT TTGGGCTTAA CAAATTGGTT AAAATGTAC                       399
(2) INFORMATIONS POUR LA SEQ ID NO: 16:
   (i) CARACTERISTIQUES DE LA SEQUENCE:
      (A) LONGUEUR: 399 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 16:
   CCCCTCTCCC CCAGGACTTT AAATGCATGG GTAAAGGCAG TAGAAGAAAA AGCCTTTAAC  60
   CCTGAGATCA TTCCTATGTT CATGGCATTG TCAGAGGGAG CTATTCCCTA TGATATTAAT 120
   ACTATGCTAA ATGCCATAGG AGGACATCAA GGAGCCCTAC AAGTGCTAAA GGAAGTAATC 180
   AATGAGGAAG CAGCAGATTG GGATAGAACT CACCCGCCAC CGATAGGGCC ATTACCACCA 240
   GGGCAGATAA GGGAACCAAC AGGAAGTGAC ATTGCTGGGA CAACTAGCAC CCAGCAAGAG 300
   CAAGTTCACT GGATTACCAG AGCCAACCAA TCTATCCCAG TAGGAGACAT CTATAGAAAA 360
   TGGATAGTGT TAGGACTAAA CAAAATGGTA AAAATGTAC                       399
(2) INFORMATIONS POUR LA SEQ ID NO: 17:
   (i) CARACTERISTIQUES DE LA SEQUENCE:
      (A) LONGUEUR: 399 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 17:
   CCCCTCTCCC CCAGGACTCT AAATGCATGG GTAAAGGCAG TAGAAGAAAA AGCCTTTAAC  60
   CCTGAAATCA TTCCTATGTT CATGGCATTG TCAGAGGGAG CAATTCCCTA TGATATTAAT 120
   ACTATGCTAA ATGCCATAGG AGGACATCAA GGAGCTTTAC AAGTGTTAAA GGAAGTAATC 180
   AATGAGGAAG CATCAGATTG GGATAGAACT CACCCACCAC CGATAGGGCC GCTGCCTCCA 240
   GGGCAAATAA GGGAACCAAC AGGAAGTGAC ATTGCTGGGA CAACTAGTAC CCAGCAAGAG 300
   CAAGTTCACT GGACTACCAG ACCCAATCAA CCTATCCCAG TAGGAGACAT CTATAGAAAA 360
   TGGATAGTGT TAGGACTAAA CAAAATGGTA AAAATGTAC                       399
(2) INFORMATIONS POUR LA SEQ ID NO: 18:
   (i) CARACTERISTIQUES DE LA SEQUENCE:
      (A) LONGUEUR: 399 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 18:
   GCCCTCTCCC CCAGGACGTT AAATGCATGG GTAAAGGCAG TAGAAGAAAA GGCCTTTAAC  60
   CCTGAAATTA TTCCTATGTT TATGGCATTA TCAGAAGGAG CTGTTCCCTA TGATATCAAT 120
   ACCATGCTAA ATGCCATAGG AGGACACCAA GGGGCTTTAC AAGTGTTGAA GGAAGTAATC 180
   AATGAGGAAG CAGCAGAATG GGATAGAACT CATCCACCAG CAATGGGGCC GTTACCACCA 240
   GGGCAGCTAA GAGATCCAAC AGGAAGTGAC ATTGCTGGAA CAACTAGCAC ACAGCAAGAG 300
   CAAATTAACT GGATTACTAG ACCAAATAAC CCTGTCCCTG TAGGAGACAT CTATAGAAAA 360
   TGGATAGTGC TAGGATTAAA TAAAATGGTA AAGTTGTAC                       399
(2) INFORMATIONS POUR LA SEQ ID NO: 19:
   (i) CARACTERISTIQUES DE LA SEQUENCE:
      (A) LONGUEUR: 399 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 19:
   GCCCTTTCCC CTAGAACTTT AAATGCATGG GTAAAGGCAG TAGAAGAAAA AGCCTTTAAC  60
   CCTGAAATCA TTCCTATGTT CATGGCATTG TCAGAGGGAG CTATTTCCTA TGACATTAAT 120
   ACTATGCTAA ATGCCATAGG AGGACATCAA GGGGCTTTAC AAGTGCTAAA GGAAGTAATC 180
   AATGAGGAAG CAGCAGAGTG GGATAGAACT CACCCAATAC CGGTAGGGCC GTTACCACCA 240
   GGGCAGATAA GGGACCCAAC AGGAAGTGAC ATTGCTGGGA CAACTAGCAC CCAGCAAGAA 300
   CAAGTTCACT GGACAACCAG ACCCAACAAC CCTATCCCAG TAGGAGACAT CTATAGGAAA 360
   TGGATAGTGT TGGGGCTTAA CAAAATGGTA AAAATGTAC                       399
(2) INFORMATIONS POUR LA SEQ ID NO: 20:
   (i) CARACTERISTIQUES DE LA SEQUENCE:
      (A) LONGUEUR: 399 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 20:
   GCTATCTCCC CCAGGACTTT AAATGCATGG GTAAAGGCAG TAGAAGAGAA GGCCTTTAAC  60
   CCTGAAATCA TTCCTATGTT CATGGCATTG TCAGAGGGAG CTATTCCCTA CGATATTAAT 120
   ACCATGCTAA ATGCCATAGG AGGACATCAA GGAGCCTTGC AGGTGCTAAA GGAAGTAATC 180
   AATGATGAAG CAGCAGATTG GGATAGAACT CACACACCAC CGGTAGGGCC GTTGCCACCA 240
   GGGCAGATAA GGGAACCAAC AGGAAGTGAC ATTGCTGGGA CAACTAGCAC CCAGCAAGAG 300
   CAAGTTCATT GGATTACTAG GCCCAACAAC CCTATCCCAG TAGGAGACAT CTATAGAAAA 360
   TGGATAGTGT TAGGGTTAAA CAAAATGGTA AAAATGTAC                       399
(2) INFORMATIONS POUR LA SEQ ID NO: 21:
   (i) CARACTERISTIQUES DE LA SEQUENCE:
      (A) LONGUEUR: 399 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 21:
   GCCCTCTCCC CCAGGACTTT AAATGCATGG GTAATAGCAG TAGAAGAGAA AGCCTTTAAC  60
   CCTGAAATTA TTCCTATGTT TATGGCATTA TCAGAAGGAG CTGTTCCCTA TGATATCAAT 120
   ACCATGCTAA ATGCCATAGG AGGACACCAG GGGGCTTTAC AAGTGTTGAA GGAAGTGATC 180
   AATGAAGAAG CAGCAGATTG GGACAGAACT CATCCACCAC CAGTAGGGCC GTTACCACCA 240
   GGTCAGATAA GGGAACCAAC AGGGAGTGAT ATTGCTGGAA CCACTAGCAC ACAGCAAGAG 300
   CAAATTCACT GGATTACTAG GGGAGGTAAT TCTATCCCAG TAGGAGACAT ATATAGGAAA 360
   TGGATAGTGC TAGGATTAAA CAAAATGGTA AAAATGTAC                       399
(2) INFORMATIONS POUR LA SEQ ID NO: 22:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "primer"
   (xi) DESCRIPTION DE LA SEQUENCE: SEQ ID NO: 22:
   AGRGAAAAAA GAGCAGTAGG AT 22
(2) INFORMATIONS POUR LA SEQ ID NO: 23:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "primer"
   (xi) DESCRIPTION DE LA SEQUENCE: SEQ ID NO: 23:
   TCTAAGTGCA GCAGGTAGCA CTAT 24
(2) INFORMATIONS POUR LA SEQ ID NO: 24:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "primer"
   (xi) DESCRIPTION DE LA SEQUENCE: SEQ ID NO: 24:
   CTAAGTTGCT CAAGAGTGGT A 21
(2) INFORMATIONS POUR LA SEQ ID NO: 25:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "primer"
   (xi) DESCRIPTION DE LA SEQUENCE: SEQ ID NO: 25:
   GTTGCTCAAG AGGTGGTAAG T                                                21
(2) INFORMATIONS POUR LA SEQ ID NO: 26:
   (i) CARACTERISTIQUES DE LA SEQUENCE:
      (A) LONGUEUR: 97 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 26:
   Val Val Thr Cys Thr His Gly Ile Lys Pro Thr Val Ser Thr Gln Leu
   1               5                   10                  15
   Ile Leu Asn Gly Thr Leu Ser Glu Gly Lys Ile Arg Met Met Ala Lys
               20                  25                  30
   Asn Ile Ser Asp Ser Gly Gln Asn Ile Ile Val Thr Leu Asn Thr Thr
           35                  40                  45
   Ile Asn Met Thr Cys Gln Arg Pro Gly His Gln Thr Val Gln Glu Ile
       50                  55                  60
   Arg Ile Gly Pro Met Ala Trp Tyr Ser Met Gly Leu Ala Asn Gly Asn
   65                  70                  75                  80
   Gly Ser Glu Ser Arg Arg Ala Tyr Cys Glu Tyr Asn Thr Thr Asn Trp
                   85                  90                  95
   Ile
(2) INFORMATIONS POUR LA SEQ ID NO: 27:
   (i) CARACTERISTIQUES DE LA SEQUENCE:
      (A) LONGUEUR: 97 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 27:
   Val Val Thr Cys Thr His Gly Ile Lys Pro Thr Val Ser Thr Gln Leu
   1               5                   10                  15
   Ile Leu Asn Gly Thr Leu Ser Glu Lys Gly Ile Arg Ile Met Gly Lys
               20                  25                  30
   Asn Ile Ser Lys Thr Gly Glu Asn Ile Ile Val Thr Leu Asn Val Ser
               35                  40                  45
   Ile Asn Ile Thr Cys His Arg Pro Gly Asn Leu Ser Val Gln Glu Met
       50                  55                  60
   Lys Ile Gly Pro Leu Ser Trp Tyr Ser Met Gly Leu Ala Ala Asn Ser
   65                  70                  75                  80
   Ser Ile Lys Ser Arg Val Ala Tyr Cys Asn Tyr Ser Thr Thr Glu Trp
                   85                  90                  95
   Thr
(2) INFORMATIONS POUR LA SEQ ID NO: 28:
   (i) CARACTERISTIQUES DE LA SEQUENCE:
      (A) LONGUEUR: 25 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 28:
   Arg Ser Val Gln Glu Met Lys Ile Gly Pro Leu Ser Trp Tyr Ser Met
   1               5                   10                  15
   Gly Leu Ala Ala Asn Ser Ser Ile Lys
               20                  25
(2) INFORMATIONS POUR LA SEQ ID NO: 29:
   (i) CARACTERISTIQUES DE LA SEQUENCE:
      (A) LONGUEUR: 98 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 29:
   Val Val Thr Cys Thr His Gly Ile Lys Pro Thr Val Ser Thr Gln Leu
   1               5                   10                  15
   Ile Met Asn Gly Thr Leu Ser Arg Gly Lys Ile Arg Ile Met Gly Arg
               20                  25                  30
   Asn Ile Thr Asp Asn Thr Lys Asn Ile Ile Val Thr Leu Asn Thr Ser
           35                  40                  45
   Ile Asn Met Thr Cys Met Arg Lys Gly Arg Gly Lys Ile Gln Arg Ile
       50                  55                  60
   Ala Thr Gly Pro Leu Arg Trp Val Ser Met Ala Ala Lys Thr Glu Ser
   65                  70                  75                  80
   Gln Asn Thr Gly Ser Arg Ile Ala Tyr Cys Met Tyr Asn Asn Thr Glu
                   85                  90                  95
   Trp Ile
(2) INFORMATIONS POUR LA SEQ ID NO: 30:
   (i) CARACTERISTIQUES DE LA SEQUENCE:
      (A) LONGUEUR: 97 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 30:
   Val Val Thr Cys Thr His Gly Ile Lys Pro Thr Val Ser Thr Gln Leu
   1               5                   10                  15
   Ile Leu Asn Gly Thr Leu Ser Lys Gly Lys Ile Arg Leu Met Ala Lys
               20                  25                  30
   Asn Ile Ser Asp Ser Gly Gln Asn Ile Ile Val Thr Leu Asn Thr Thr
           35                  40                  45
   Ile Asn Met Thr Cys His Arg Pro Gly Asn Leu Lys Val Gln Glu Ile
       50                  55                  60
   Lys Ile Gly Pro Met Ala Trp Tyr Ser Met Gly Ile Glu Ala Glu Asn
   65                  70                  75                  80
   Ile Pro Asp Ser Arg Lys Ala Tyr Cys Asp Tyr Asn Ala Thr Lys Trp
                   85                  90                  95
   Val
(2) INFORMATIONS POUR LA SEQ ID NO: 31:
   (i) CARACTERISTIQUES DE LA SEQUENCE:
      (A) LONGUEUR: 99 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 31:
   Val Val Thr Cys Thr His Gly Ile Lys Pro Ala Val Ser Thr Gln Leu
   1               5                   10                  15
   Ile Leu Asn Gly Thr Leu Ser Glu Gly Lys Ile Arg Ile Met Gly Gln
               20                  25                  30
   Asn Ile Ser Asp Ser Gly Lys Asn Ile Ile Val Thr Leu Asn Lys Thr
           35                  40                  45
   Val Asn Met Asn Ile Thr Cys Thr Arg Asp Gly Asp Gln Lys Val Gln
       50                  55                  60
   Glu Ile Gly Ile Gly Pro Leu Ser Trp Tyr Ser Met Ser Ile Ala Glu
   65                  70                  75                  80
   Asp Ser Ala Lys Asn Thr Arg Ala Ala Tyr Cys Asn Tyr Ser Ala Ser
                   85                  90                  95
   Ser Trp Lys
(2) INFORMATIONS POUR LA SEQ ID NO: 32:
   (i) CARACTERISTIQUES DE LA SEQUENCE:
      (A) LONGUEUR: 98 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 32:
   Val Val Thr Cys Thr His Gly Ile Lys Pro Thr Val Ser Thr His Leu
   1               5                   10                  15
   Ile Leu Asn Gly Thr Ile Ser Glu Gly Glu Ile Arg Ile Met Gly Lys
               20                  25                  30
   Asn Ile Arg Glu Asn Ala Lys Asn Ile Ile Val Thr Leu Asn Ser Thr
           35                  40                  45
   Ile Asn Met Thr Cys Glu Arg Pro Glu Gly Asn Leu Thr Ile Gln Glu
       50                  55                  60
   Ile His Ser Gly Pro Met Ala Trp Tyr Ser Leu Gly Leu Lys Arg Asn
   65                  70                  75                  80
   Thr Thr Val Arg Ser Arg Ser Ala His Cys Lys Tyr Asn Thr Thr Asn
                   85                  90                  95
   Trp Glu
(2) INFORMATIONS POUR LA SEQ ID NO: 33:
   (i) CARACTERISTIQUES DE LA SEQUENCE:
      (A) LONGUEUR: 25 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 33:
   Arg Thr Ile Gln Glu Ile His Ser Gly Pro Met Ala Trp Tyr Ser Leu
   1               5                   10                  15
   Gly Leu Lys Arg Asn Thr Thr Val Arg
               20                  25
(2) INFORMATIONS POUR LA SEQ ID NO: 34:
   (i) CARACTERISTIQUES DE LA SEQUENCE:
      (A) LONGUEUR: 99 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 34:
   Val Val Thr Cys Thr His Gly Ile Lys Pro Ala Val Ser Thr Gln Leu
   1               5                   10                  15
   Ile Leu Asn Gly Thr Leu Ser Lys Gly Lys Ile Arg Ile Met Ala Lys
               20                  25                  30
   Asn Ile Thr Asn Thr Gly Asn Asn Ile Ile Val Thr Leu Asn Ser Thr
           35                  40                  45
   Ile Asn Ile Thr Cys Asn Arg Pro Gly Arg Gly Ile Lys Gln Ile Gly
       50                  55                  60
   Ile Gly Pro Met Ser Val Tyr Ser Gly Ser Leu Ala Asp Leu Gly Gly
   65                  70                  75                  80
   Asn Asn Asn Ser Arg Ile Ala Tyr Cys Asp Tyr Asp Ile Thr Lys Trp
                   85                  90                  95
   Asn Glu Thr
(2) INFORMATIONS POUR LA SEQ ID NO: 35:
   (i) CARACTERISTIQUES DE LA SEQUENCE:
      (A) LONGUEUR: 24 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 35:
   Arg Ile Lys Gln Ile Gly Ile Gly Pro Met Ser Val Tyr Ser Gly Ser
   1               5                   10                  15
   Leu Ala Asp Leu Gly Asn Asn Asn
               20
(2) INFORMATIONS POUR LA SEQ ID NO: 36:
   (i) CARACTERISTIQUES DE LA SEQUENCE:
      (A) LONGUEUR: 40 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 36:
   Arg Gln Leu Arg Ala Arg Leu Leu Ala Leu Glu Thr Leu Ile Gln Asn
   1               5                   10                  15
   Gln Gln Leu Leu Asn Ser Trp Gly Cys Lys Gly Arg Ile Val Cys Tyr
               20                  25                  30
   Thr Ser Val Lys Trp Asn Trp Thr
           35                  40
(2) INFORMATIONS POUR LA SEQ ID NO: 37:
   (i) CARACTERISTIQUES DE LA SEQUENCE:
      (A) LONGUEUR: 40 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 37:
   Arg Gln Leu Arg Ala Arg Leu Leu Ala Leu Glu Thr Leu Ile Gln Asn
   1               5                   10                  15
   Gln Gln Leu Leu Asn Leu Trp Gly Cys Lys Gly Arg Leu Leu Cys Tyr
               20                  25                  30
   Thr Ser Val Lys Trp Asn Ser Thr
           35                  40
(2) INFORMATIONS POUR LA SEQ ID NO: 38:
   (i) CARACTERISTIQUES DE LA SEQUENCE:
      (A) LONGUEUR: 40 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 38:
   Arg Gln Leu Arg Ala Arg Leu Gln Ala Leu Glu Pro Leu Ile Gln Asn
   1               5                   10                  15
   Gln Gln Arg Leu Ser Leu Trp Gly Cys Lys Gly Arg Ile Ile Cys Tyr
               20                  25                  30
   Thr Ser Ala Lys Trp Asn Asn Thr
           35                  40
(2) INFORMATIONS POUR LA SEQ ID NO: 39:
   (i) CARACTERISTIQUES DE LA SEQUENCE:
      (A) LONGUEUR: 40 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 39:
   Arg Gln Leu Arg Ala Arg Leu Leu Ala Leu Glu Thr Leu Ile Gln Asn
   1               5                   10                  15
   Gln Gln Leu Leu Asn Ser Trp Gly Cys Lys Gly Arg Leu Val Cys Tyr
               20                  25                  30
   Thr Ser Val Lys Trp Asn Glu Thr
           35                  40
(2) INFORMATIONS POUR LA SEQ ID NO: 40:
   (i) CARACTERISTIQUES DE LA SEQUENCE:
      (A) LONGUEUR: 40 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 40:
   Arg Gln Leu Arg Ala Arg Leu Leu Ala Leu Glu Thr Leu Ile Gln Asn
   1               5                   10                  15
   Gln Gln Leu Leu Asn Leu Trp Gly Cys Lys Gly Arg Leu Leu Cys Tyr
               20                  25                  30
   Thr Ser Val Lys Trp Asn Thr Thr
           35                  40
(2) INFORMATIONS POUR LA SEQ ID NO: 41:
   (i) CARACTERISTIQUES DE LA SEQUENCE:
      (A) LONGUEUR: 40 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 41:
   Arg Gln Leu Arg Ala Arg Leu Gln Ala Leu Glu Thr Leu Ile Gln Asn
   1               5                   10                  15
   Gln Gln Leu Leu Ser Leu Trp Gly Cys Lys Gly Arg Leu Val Cys Tyr
               20                  25                  30
   Thr Ser Val Lys Trp His Asn Thr
           35                  40
(2) INFORMATIONS POUR LA SEQ ID NO: 42:
   (i) CARACTERISTIQUES DE LA SEQUENCE:
      (A) LONGUEUR: 40 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 42:
   Arg Gln Leu Arg Ala Arg Leu Val Ala Leu Glu Thr Leu Val Gln Asn
   1               5                   10                  15
   Gln Gln Leu Leu Asn Leu Trp Gly Cys Lys Gly Arg Leu Thr Cys Tyr
               20                  25                  30
   Thr Ser Val Lys Trp Asn Asp Thr
           35                  40
(2) INFORMATIONS POUR LA SEQ ID NO: 43:
   (i) CARACTERISTIQUES DE LA SEQUENCE:
      (A) LONGUEUR: 133 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 43:
   Pro Ile Ser Pro Arg Thr Leu Asn Ala Trp Val Lys Ala Val Glu Glu
   1               5                   10                  15
   Lys Ala Phe Asn Pro Glu Ile Ile Pro Met Phe Met Ala Leu Ser Glu
               20                  25                  30
   Gly Ala Val Pro Tyr Asp Ile Asn Thr Met Leu Asn Ala Ile Gly Glu
           35                  40                  45
   His Gln Gly Ala Leu Gln Val Leu Lys Glu Val Ile Asn Glu Glu Ala
       50                  55                  60
   Leu Glu Trp Asp Arg Thr His Pro Pro Pro Ile Gly Pro Leu Pro Pro
   65                  70                  75                  80
   Gly Gln Ile Arg Asp Pro Thr Gly Ser Asp Ile Ala Gly Thr Thr Ser
                   85                  90                  95
   Thr Gln Gln Glu Gln Val His Trp Val Thr Arg Asn Pro Asn Pro Ile
               100                 105                 110
   Pro Val Gly Asp Ile Tyr Trp Lys Trp Ile Val Phe Gly Leu Asn Lys
           115                 120                 125
   Leu Val Lys Met Tyr
       130
(2) INFORMATIONS POUR LA SEQ ID NO: 44:
   (i) CARACTERISTIQUES DE LA SEQUENCE:
      (A) LONGUEUR: 133 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 44:
   Ala Ile Ser Pro Arg Thr Leu Asn Ala Trp Val Lys Ala Val Glu Glu
   1               5                   10                  15
   Lys Ala Phe Asn Pro Glu Ile Ile Pro Met Phe Met Ala Leu Ser Glu
               20                  25                  30
   Gly Ala Ile Pro Tyr Asp Ile Asn Thr Met Leu Asn Ala Ile Gly Gly
           35                  40                  45
   His Gln Gly Ala Leu Gln Val Leu Lys Glu Val Ile Asn Asp Glu Ala
       50                  55                  60
   Ala Asp Trp Asp Arg Thr His Thr Pro Pro Val Gly Pro Leu Pro Pro
   65                  70                  75                  80
   Gly Gln Ile Arg Glu Pro Thr Gly Ser Asp Ile Ala Gly Thr Thr Ser
                   85                  90                  95
   Thr Gln Gln Glu Gln Val His Trp Ile Thr Arg Pro Asn Asn Pro Ile
               100                 105                 110
   Pro Val Gly Asp Ile Tyr Arg Lys Trp Ile Val Leu Gly Leu Asn Lys
           115                 120                 125
   Met Val Lys Met Tyr
       130
(2) INFORMATIONS POUR LA SEQ ID NO: 45:
   (i) CARACTERISTIQUES DE LA SEQUENCE:
      (A) LONGUEUR: 133 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 45:
   Ala Leu Ser Pro Arg Thr Leu Asn Ala Trp Val Lys Ala Val Glu Glu
   1               5                   10                  15
   Lys Ala Phe Asn Pro Glu Ile Ile Pro Met Phe Met Ala Leu Ser Glu
               20                  25                  30
   Gly Ala Val Pro Tyr Asp Ile Asn Thr Met Leu Asn Ala Ile Gly Gly
           35                  40                  45
   His Gln Gly Ala Leu Gln Val Leu Lys Glu Val Ile Asn Glu Glu Ala
       50                  55                  60
   Ala Glu Trp Asp Arg Thr His Pro Pro Ala Met Gly Pro Leu Pro Pro
   65                  70                  75                  80
   Gly Gln Ile Arg Asp Pro Thr Gly Ser Asp Ile Ala Gly Thr Thr Ser
                   85                  90                  95
   Thr Gln Gln Glu Gln Ile Asn Trp Ile Thr Arg Pro Asn Asn Pro Val
               100                 105                 110
   Pro Val Gly Asp Ile Tyr Arg Lys Trp Ile Val Leu Gly Leu Asn Lys
           115                 120                 125
   Met Val Lys Leu Tyr
       130
(2) INFORMATIONS POUR LA SEQ ID NO: 46:
   (i) CARACTERISTIQUES DE LA SEQUENCE:
      (A) LONGUEUR: 133 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 46:
   Ala Leu Ser Pro Arg Thr Leu Asn Ala Trp Val Lys Ala Val Glu Glu
   1               5                   10                  15
   Lys Ala Phe Asn Pro Glu Ile Ile Pro Met Phe Met Ala Leu Ser Glu
               20                  25                  30
   Gly Ala Ile Ser Tyr Asp Ile Asn Thr Met Leu Asn Ala Ile Gly Gly
           35                  40                  45
   His Gln Gly Ala Leu Gln Val Leu Lys Glu Val Ile Asn Glu Glu Ala
       50                  55                  60
   Ala Glu Trp Asp Arg Thr His Pro Ile Pro Val Gly Pro Leu Pro Pro
   65                  70                  75                  80
   Gly Gln Ile Arg Asp Pro Thr Gly Ser Asp Ile Ala Gly Thr Thr Ser
                   85                  90                  95
   Thr Gln Gln Glu Gln Val His Trp Thr Thr Arg Pro Asn Asn Pro Ile
               100                 105                 110
   Pro Val Gly Asp Ile Tyr Arg Lys Trp Ile Val Leu Gly Leu Asn Lys
           115                 120                 125
   Met Val Lys Met Tyr
       130
(2) INFORMATIONS POUR LA SEQ ID NO: 47:
   (i) CARACTERISTIQUES DE LA SEQUENCE:
      (A) LONGUEUR: 133 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 47:
   Ala Leu Ser Pro Arg Thr Leu Asn Ala Trp Val Ile Ala Val Glu Glu
   1               5                   10                  15
   Lys Ala Phe Asn Pro Glu Ile Ile Pro Met Phe Met Ala Leu Ser Glu
               20                  25                  30
   Gly Ala Val Pro Tyr Asp Ile Asn Thr Met Leu Asn Ala Ile Gly Gly
           35                  40                  45
   His Gln Gly Ala Leu Gln Val Leu Lys Glu Val Ile Asn Glu Glu Ala
       50                  55                  60
   Ala Asp Trp Asp Arg Thr His Pro Pro Pro Val Gly Pro Leu Pro Pro
   65                  70                  75                  80
   Gly Gln Ile Arg Glu Pro Thr Gly Ser Asp Ile Ala Gly Thr Thr Ser
                   85                  90                  95
   Thr Gln Gln Glu Gln Ile His Trp Ile Thr Arg Gly Gly Asn Ser Ile
               100                 105                 110
   Pro Val Gly Asp Ile Tyr Arg Lys Trp Ile Val Leu Gly Leu Asn Lys
           115                 120                 125
   Met Val Lys Met Tyr
       130
(2) INFORMATIONS POUR LA SEQ ID NO: 48:
   (i) CARACTERISTIQUES DE LA SEQUENCE:
      (A) LONGUEUR: 133 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 48:
   Pro Leu Ser Pro Arg Thr Leu Asn Ala Trp Val Lys Ala Val Glu Glu
   1               5                   10                  15
   Lys Ala Phe Asn Pro Glu Ile Ile Pro Met Phe Met Ala Leu Ser Glu
               20                  25                  30
   Gly Ala Ile Pro Tyr Asp Ile Asn Thr Met Leu Asn Ala Ile Gly Gly
           35                  40                  45
   His Gln Gly Ala Leu Gln Val Leu Lys Glu Val Ile Asn Glu Glu Ala
       50                  55                  60
   Ala Asp Trp Asp Arg Thr His Pro Pro Pro Ile Gly Pro Leu Pro Pro
   65                  70                  75                  80
   Gly Gln Ile Arg Glu Pro Thr Gly Ser Asp Ile Ala Gly Thr Thr Ser
                   85                  90                  95
   Thr Gln Gln Glu Gln Val His Trp Ile Thr Arg Ala Asn Gln Ser Ile
               100                 105                 110
   Pro Val Gly Asp Ile Tyr Arg Lys Trp Ile Val Leu Gly Leu Asn Lys
           115                 120                     125
   Met Val Lys Met Tyr
       130
(2) INFORMATIONS POUR LA SEQ ID NO: 49:
   (i) CARACTERISTIQUES DE LA SEQUENCE:
      (A) LONGUEUR: 133 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 49:
   Pro Leu Ser Pro Arg Thr Leu Asn Ala Trp Val Lys Ala Val Glu Glu
   1               5                   10                  15
   Lys Ala Phe Asn Pro Glu Ile Ile Pro Met Phe Met Ala Leu Ser Glu
               20                  25                  30
   Gly Ala Ile Pro Tyr Asp Ile Asn Thr Met Leu Asn Ala Ile Gly Gly
           35                  40                  45
   His Gln Gly Ala Leu Gln Val Leu Lys Glu Val Ile Asn Glu Glu Ala
       50                  55                  60
   Ser Asp Trp Asp Arg Thr His Pro Pro Pro Ile Gly Pro Leu Pro Pro
   65                  70                  75                  80
   Gly Gln Ile Arg Glu Pro Thr Gly Ser Asp Ile Ala Gly Thr Thr Ser
                   85                  90                  95
   Thr Gln Gln Glu Gln Val His Trp Thr Thr Arg Pro Asn Gln Pro Ile
               100                 105                 110
   Pro Val Gly Asp Ile Tyr Arg Lys Trp Ile Val Leu Gly Leu Asn Lys
           115                 120                 125
   Met Val Lys Met Tyr
       130
(2) INFORMATIONS POUR LA SEQ ID NO: 50:
   (i) CARACTERISTIQUES DE LA SEQUENCE:
      (A) LONGUEUR: 282 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 50:
   TGTACACATG GCATCAAACC AACAGTGAGT ACTCACCTAA TATTAAATGG GACACTCTCT  60
   GAAGGAAAAA TAAGAATTAT GGGAAAAAAT ATCTCGGACA CTGGGAAAAA TATCATAGTG 120
   ACCCTAAATT CTACTATAAA CATAACCTGT GTGAGACCAT GGAATCAGAC AGTACAAACG 180
   ATAGGAATAG GACCAATGTC CTGGCTCAGC ATGGACATAA ATGCAGATAA AAACAATAAC 240
   TCAAGAATAG CTTATTGCGA GTATAACACC ACGGATTGGG AA                    282
(2) INFORMATIONS POUR LA SEQ ID NO: 51:
   (i) CARACTERISTIQUES DE LA SEQUENCE:
      (A) LONGUEUR: 279 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 51:
   TGTACACATG GCATCAAGCC CACAGTGAGC ACCCACCTGA TATTAAATGG GACACTCTCT  60
   GAAGGAAAAA TAAGAATTAT GGGAAAAAAC ATTTCAGATA ATGCGAAAAA TATCATAGTG 120
   ACCCTAAAAC AGACTATAAG CATAACTTGT GAGAGACCAG GAAATCTTTC AGTACAAGAG 180
   ATAAAAATAG GTCCAATGGC CTGGTACAGC ATGGCCGTAG AGCAAGATAA GTCAACCTCC 240
   AGGACAGCTT ATTGCAAGTA TAATGTCACT AAGTGGAAA                        279
(2) INFORMATIONS POUR LA SEQ ID NO: 52:
   (i) CARACTERISTIQUES DE LA SEQUENCE:
      (A) LONGUEUR: 282 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 52:
   TGTACACATG GCATCAAGCC AACAGTAAGT ACTCAGTTAA TATTAAATGG AACACTCTCG  60
   GAAGGAAAGA TAAGAATAAT GGCAAAAGAT ATTTTAAATA GTGGCAAAAA TATCATAGTG 120
   ACCCTAAATA CTACTGTAAA CATGACCTGC GTGAGACCAG GAAATATAAC AATACAAACG 180
   TTAAAGATAG GTCCACTGGC CTGGTACAGC ATGGACATAG CGAATGAAAA AGACCATAAG 240
   TCAAGAACAG CTTATTGTGA GTATAATACC ACTAATTGGG TA                    282
(2) INFORMATIONS POUR LA SEQ ID NO: 53:
   (i) CARACTERISTIQUES DE LA SEQUENCE:
      (A) LONGUEUR: 279 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 53:
   TGTACACATG GCATCAAGCC AACAGTAAGT ACTCAGCTAA TATTAAATAG AACACTCTCG  60
   GAAGGAAAGA TAAAAATAAT GACAAAAAAT ATTTCGGAGA ATGGAAATAT TATAGTGACC 120
   CTAAATACTA CTATAAACAT GACCTGCGAG AGACCAGGAA ATCTATCAGT ACAAGAGATA 180
   AACATAGGTC CACTGGCCTG GTACAGCATG AGCATAAAGA ATGAAGGAAA AACTGAGTCA 240
   AGAGTAGCTT ATTGTGAGTA TAACAGCACT AATTGGGTA                        279
(2) INFORMATIONS POUR LA SEQ ID NO: 54:
   (i) CARACTERISTIQUES DE LA SEQUENCE:
      (A) LONGUEUR: 120 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 54:
   AGACAACTCC GAGCTCGCCT GCTAGCCTTA GAAACCTTAA TACAGAATCA GCAACTCCTA  60
   AACCTATGGG GCTGTAAGGG AAGGCTGGTC TGTTACACAT CAGTAAAATG GAACATGTCA 120
(2) INFORMATIONS POUR LA SEQ ID NO: 55:
   (i) CARACTERISTIQUES DE LA SEQUENCE:
      (A) LONGUEUR: 120 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 55:
(2) INFORMATIONS POUR LA SEQ ID NO: 56:
   (i) CARACTERISTIQUES DE LA SEQUENCE:
      (A) LONGUEUR: 119 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 56:
   AGACAACTCC GAGCTCGCCT GCTAGCCTTA GAAACCTTAA TACAGAATCA GCAACTCCTA  60
   AACTCGTGGG GCTGTTGGGA AGACTAGTCT GTTACACATC AGTAGAATGG AACTGGACA  119
(2) INFORMATIONS POUR LA SEQ ID NO: 57:
   (i) CARACTERISTIQUES DE LA SEQUENCE:
      (A) LONGUEUR: 120 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 57:
   AGACAACTCC GAGCTCGCCT GCTAGCCTTA GAAACCTTAA TTCAGAATCA GCAACTCCTA  60
   AACTCGTGGG GCTGTAAGGG AAGACAAGTC TGTTACACAT CAGTAAAATG GAACAATACA 120
(2) INFORMATIONS POUR LA SEQ ID NO: 58:
   (i) CARACTERISTIQUES DE LA SEQUENCE:
      (A) LONGUEUR: 399 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 58:
   GCCCTCTCCC CCAGGACTTT AAATGCATGG GTAAAGGCAG TAGAAGAAAA GGCCTTTAAC  60
   CCGGAAATCA TTCCTATGTT CATGGCATTG TCAGAGGGAG CTGTTCCCTA TGATATTAAT 120
   ACTATGCTAA ATGCCATAGG AGGACATCAA GGAGCATTAC AAGTGCTAAA AGAAGTAATC 180
   AATGAGGAAG CAGCAGAGTG GGATAGAACT CACCCACAAG CAGTAGGGCC ATTGCCACCA 240
   GGACAGATAA GGGAACCAAC AGGAAGTGAC ATTGCTGGAA CAACCAGTAC CCAGCAAGAG 300
   CAAATTCACT GGACTACCAG GGCCAACCCC CCTATCCCAG TAGGAGACAT CTATAGAAAA 360
   TGGATAGTGT TAGGGCTAAA CAAAATGGTA AAAATGTAC                        399
(2) INFORMATIONS POUR LA SEQ ID NO: 59:
   (i) CARACTERISTIQUES DE LA SEQUENCE:
      (A) LONGUEUR: 399 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 59:
   GCCCTCTCCC CCAGGACTTT AAATGCATGG GTAAAGGCAG TAGAAGAAAA GGCCTTTAAC  60
   CCTGAAATCA TTCCTATGTT CATGGCATTA TCAGAGGGAG CTATTTCCTA TGATATTAAT 120
   ACCATGCTAA ATGCCATAGG AGGACATCAA GGGGCTCTAC AGGTGCTAAA GGAAGTAATC 180
   AATGAAGAAG CAGCAGATTG GGATAGAGCT CACCCACCAG TGGTAGGGCC GTTGGCACCA 240
   GGGCAGATGA GGGACCCAAC AGGAAGTGAC ATCGCTGGGA CAACTAGCAC CCAGCAAGAG 300
   CAAATTCATT GGACTACCAG GCCCAACAAC CCTATCCCAG TAGGAGACAT CTATAGAAAA 360
   TGGATAGTGT TAGGACTAAA CAAAATGGTA AAAATGTAC                        399
(2) INFORMATIONS POUR LA SEQ ID NO: 60:
   (i) CARACTERISTIQUES DE LA SEQUENCE:
      (A) LONGUEUR: 397 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 60:
   GCCATTTCCC CTAGGACTTT AAATGCATGG GTAAAGGCAG TAGAAGAAAA AGCCTTTAAC  60
   CCTAAAATCA TTCCTATGTT CATGGCATTG TCAGAGGGAG CTGTTCCCTA TGATATTAAT 120
   ACTATGCTAA ATGCCATAGG AGGACATCAA GGGGCTTTAC AAGTGCTAAA GGAAGTAATC 180
   AATGAGGAAG CATCGGAGTG GGATAGAACT CACCCACCAC CGATAGGGCC GTTACCACCA 240
   GGCAGATAAG GGACCCAACA GGAAGTGACA TTGCTGGACA ACTAGCACCC AGCAAGAGCA 300
   AGTTCACTGG ATTACCAGGG CCCCCAACCC TATCCCAGTA GGAGACATCT ATAGAAAATG 360
   GATAGTGTTG GGACTAAACA AAATGGTAAA AATGTAC                          397
(2) INFORMATIONS POUR LA SEQ ID NO: 61:
   (i) CARACTERISTIQUES DE LA SEQUENCE:
      (A) LONGUEUR: 399 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 61:
   GCCATTTCCC CTAGGACTCT AAATGCATGG GTAAAGGCAG TAGAAGAAAA GGCCTTTAAC  60
   CCTGAAATCA TTCCTATGTT CATGGCATTG TCAGAGGGAG CTATTCCCTA TGATATTAAT 120
   ACCATGCTAA ATGCCATAGG AGGACATCAA GGGGCTTTAC AAGTGCTAAA GGAAGTAATC 180
   AATGAGGAAG CATCAGAATG GGATAGAACT CACCCACAAC AGGCAGGGCC GTTACCACCA 240
   GGGCAGATAA GGGACCCAAC AGGAAGTGAC ATTGCTGGGA CAACTAGCAC CCAGCAAGAG 300
   CAAGTTCACT GGACTACCAG GGCCGCCAAC CCTATCCCAG TAGGAGACAT CTATAGAAAA 360
   TGGATAGTGT TGGGACTAAT CAAAATGGTA AAAATGTAC                        399
(2) INFORMATIONS POUR LA SEQ ID NO: 62:
   (i) CARACTERISTIQUES DE LA SEQUENCE:
      (A) LONGUEUR: 94 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 62:
   Cys Thr His Gly Ile Lys Pro Thr Val Ser Thr His Leu Ile Leu Asn
   1               5                   10                  15
   Gly Thr Leu Ser Glu Gly Lys Ile Arg Ile Met Gly Lys Asn Ile Ser
               20                  25                  30
   Asp Thr Gly Lys Asn Ile Ile Val Thr Leu Asn Ser Thr Ile Asn Ile
           35                  40                  45
   Thr Cys Val Arg Pro Trp Asn Gln Thr Val Gln Thr Ile Gly Ile Gly
       50                  55                  60
   Pro Met Ser Trp Leu Ser Met Asp Ile Asn Ala Asp Lys Asn Asn Asn
   65                  70                  75                  80
   Ser Arg Ile Ala Tyr Cys Glu Tyr Asn Thr Thr Asp Trp Glu
                   85                  90
(2) INFORMATIONS POUR LA SEQ ID NO: 63:
   (i) CARACTERISTIQUES DE LA SEQUENCE:
      (A) LONGUEUR: 93 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 63:
   Cys Thr His Gly Ile Lys Pro Thr Val Ser Thr His Leu Ile Leu Asn
   1               5                   10                  15
   Gly Thr Leu Ser Glu Gly Lys Ile Arg Ile Met Gly Lys Asn Ile Ser
               20                  25                  30
   Asp Asn Ala Lys Asn Ile Ile Val Thr Leu Lys Gln Thr Ile Ser Ile
           35                  40                  45
   Thr Cys Glu Arg Pro Gly Asn Leu Ser Val Gln Glu Ile Lys Ile Gly
       50                  55                  60
   Pro Met Ala Trp Tyr Ser Met Ala Val Glu Gln Asp Lys Ser Thr Ser
   65                  70                  75                  80
   Arg Thr Ala Tyr Cys Lys Tyr Asn Val Thr Lys Trp Lys
                   85                  90
(2) INFORMATIONS POUR LA SEQ ID NO: 64:
   (i) CARACTERISTIQUES DE LA SEQUENCE:
      (A) LONGUEUR: 94 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 64:
   Cys Thr His Gly Ile Lys Pro Thr Val Ser Thr Gln Leu Ile Leu Asn
   1               5                   10                  15
   Gly Thr Leu Ser Glu Gly Lys Ile Arg Ile Met Ala Lys Asp Ile Leu
               20                  25                  30
   Asn Ser Gly Lys Asn Ile Ile Val Thr Leu Asn Thr Thr Val Asn Met
           35                  40                  45
   Thr Cys Val Arg Pro Gly Asn Ile Thr Ile Gln Thr Leu Lys Ile Gly
       50                  55                  60
   Pro Leu Ala Trp Tyr Ser Met Asp Ile Ala Asn Glu Lys Asp His Lys
   65                  70                  75                  80
   Ser Arg Thr Ala Tyr Cys Glu Tyr Asn Thr Thr Asn Trp Val
               85                  90
(2) INFORMATIONS POUR LA SEQ ID NO: 65:
   (i) CARACTERISTIQUES DE LA SEQUENCE:
      (A) LONGUEUR: 93 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 65:
   Cys Thr His Gly Ile Lys Pro Thr Val Ser Thr Gln Leu Ile Leu Asn
   1               5                   10                  15
   Arg Thr Leu Ser Glu Gly Lys Ile Lys Ile Met Thr Lys Asn Ile Ser
               20                  25                  30
   Glu Asn Gly Asn Ile Ile Val Thr Leu Asn Thr Thr Ile Asn Met Thr
           35                  40                  45
   Cys Glu Arg Pro Gly Asn Leu Ser Val Gln Glu Ile Asn Ile Gly Pro
       50                  55                  60
   Leu Ala Trp Tyr Ser Met Ser Ile Lys Asn Glu Gly Lys Thr Glu Ser
   65                  70                  75                  80
   Arg Val Ala Tyr Cys Glu Tyr Asn Ser Thr Asn Trp Val
                   85                  90
(2) INFORMATIONS POUR LA SEQ ID NO: 66:
   (i) CARACTERISTIQUES DE LA SEQUENCE:
      (A) LONGUEUR: 42 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 66:
   Arg Gln Leu Arg Ala Arg Leu Leu Ala Leu Glu Thr Leu Ile Gln Asn
   1               5                   10                  15
   Gln Gln Leu Leu Asn Leu Trp Gly Cys Lys Gly Arg Leu Val Cys Tyr
               20                  25                  30
   Thr Ser Val Lys Trp Asn Met Ser Trp Ala
           35                  40
(2) INFORMATIONS POUR LA SEQ ID NO: 67:
   (i) CARACTERISTIQUES DE LA SEQUENCE:
      (A) LONGUEUR: 41 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 67:
   Arg Gln Leu Arg Ala Arg Leu Leu Ala Leu Glu Thr Leu Ile Gln Asn
   1               5                   10                  15
   Gln Gln Leu Leu Asn Leu Trp Gly Cys Lys Gly Arg Leu Ile Cys Tyr
               20                  25                  30
   Thr Ser Val Lys Trp Asn Ser Thr Trp
           35                  40
(2) INFORMATIONS POUR LA SEQ ID NO: 68:
   (i) CARACTERISTIQUES DE LA SEQUENCE:
      (A) LONGUEUR: 40 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 68:
   Arg Gln Leu Arg Ala Arg Leu Leu Ala Leu Glu Thr Leu Ile Gln Asn
   1               5                   10                  15
   Gln Gln Leu Leu Asn Ser Trp Gly Cys Lys Gly Arg Leu Val Cys Tyr
               20                  25                  30
   Thr Ser Val Glu Trp Asn Trp Thr
           35                  40
(2) INFORMATIONS POUR LA SEQ ID NO: 69:
   (i) CARACTERISTIQUES DE LA SEQUENCE:
      (A) LONGUEUR: 41 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 69:
   Arg Gln Leu Arg Ala Arg Leu Leu Ala Leu Glu Thr Leu Ile Gln Asn
   1               5                   10                  15
   Gln Gln Leu Leu Asn Ser Trp Gly Cys Lys Gly Arg Gln Val Cys Tyr
               20                  25                  30
   Thr Ser Val Lys Trp Asn Asn Thr Trp
           35                  40
(2) INFORMATIONS POUR LA SEQ ID NO: 70:
   (i) CARACTERISTIQUES DE LA SEQUENCE:
      (A) LONGUEUR: 133 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 70:
   Ala Leu Ser Pro Arg Thr Leu Asn Ala Trp Val Lys Ala Val Glu Glu
   1               5                   10                  15
   Lys Ala Phe Asn Pro Glu Ile Ile Pro Met Phe Met Ala Leu Ser Glu
               20                  25                  30
   Gly Ala Val Pro Tyr Asp Ile Asn Thr Met Leu Asn Ala Ile Gly Gly
           35                  40                  45
   His Gln Gly Ala Leu Gln Val Leu Lys Glu Val Ile Asn Glu Glu Ala
       50                  55                  60
   Ala Glu Trp Asp Arg Thr His Pro Gln Ala Val Gly Pro Leu Pro Pro
   65                  70                  75                  80
   Gly Gln Ile Arg Glu Pro Thr Gly Ser Asp Ile Ala Gly Thr Thr Ser
                   85                  90                  95
   Thr Gln Gln Glu Gln Ile His Trp Thr Thr Arg Ala Asn Pro Pro Ile
               100                 105                 110
   Pro Val Gly Asp Ile Tyr Arg Lys Trp Ile Val Leu Gly Leu Asn Lys
           115                 120                 125
   Met Val Lys Met Tyr
       130
(2) INFORMATIONS POUR LA SEQ ID NO: 71:
   (i) CARACTERISTIQUES DE LA SEQUENCE:
      (A) LONGUEUR: 133 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 71:
   Ala Leu Ser Pro Arg Thr Leu Asn Ala Trp Val Lys Ala Val Glu Glu
   1               5                   10                  15
   Lys Ala Phe Asn Pro Glu Ile Ile Pro Met Phe Met Ala Leu Ser Glu
               20                  25                  30
   Gly Ala Ile Ser Tyr Asp Ile Asn Thr Met Leu Asn Ala Ile Gly Gly
           35                  40                  45
   His Gln Gly Ala Leu Gln Val Leu Lys Glu Val Ile Asn Glu Glu Ala
       50                  55                  60
   Ala Asp Trp Asp Arg Ala His Pro Pro Val Val Gly Pro Leu Ala Pro
   65 70 75 80
   Gly Gln Met Arg Asp Pro Thr Gly Ser Asp Ile Ala Gly Thr Thr Ser
   85 90 95
   Thr Gln Gln Glu Gln Ile His Trp Thr Thr Arg Pro Asn Asn Pro Ile
   100 105 110
   Pro Val Gly Asp Ile Tyr Arg Lys Trp Ile Val Leu Gly Leu Asn Lys
   115 120 125
   Met Val Lys Met Tyr
   130
(2) INFORMATIONS POUR LA SEQ ID NO: 72:
   (i) CARACTERISTIQUES DE LA SEQUENCE:
      (A) LONGUEUR: 133 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 72:
   Ala Ile Ser Pro Arg Thr Leu Asn Ala Trp Val Lys Ala Val Glu Glu
   1 5 10 15
   Lys Ala Phe Asn Pro Glu Ile Ile Pro Met Phe Met Ala Leu Ser Glu
   20 25 30
   Gly Ala Val Pro Tyr Asp Ile Asn Thr Met Leu Asn Ala Ile Gly Gly
   35 40 45
   His Gln Gly Ala Leu Gln Val Leu Lys Glu Val Ile Asn Glu Glu Ala
   50 55 60
   Ser Glu Trp Asp Arg Thr His Pro Pro Pro Ile Gly Pro Leu Pro Pro
   65 70 75 80
   Gly Gln Ile Arg Asp Pro Thr Gly Ser Asp Ile Ala Gly Thr Thr Ser
   85 90 95
   Thr Gln Gln Glu Gln Val His Trp Ile Thr Arg Ala Pro Asn Pro Ile
   100 105 110
   Pro Val Gly Asp Ile Tyr Arg Lys Trp Ile Val Leu Gly Leu Asn Lys
   115 120 125
   Met Val Lys Met Tyr
   130
(2) INFORMATIONS POUR LA SEQ ID NO: 73:
   (i) CARACTERISTIQUES DE LA SEQUENCE:
      (A) LONGUEUR: 133 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 73:
   Ala Ile Ser Pro Arg Thr Leu Asn Ala Trp Val Lys Ala Val Glu Glu
   1               5                   10                  15
   Lys Ala Phe Asn Pro Glu Ile Ile Pro Met Phe Met Ala Leu Ser Glu
               20                  25                  30
   Gly Ala Ile Pro Tyr Asp Ile Asn Thr Met Leu Asn Ala Ile Gly Gly
           35                  40                  45
   His Gln Gly Ala Leu Gln Val Leu Lys Glu Val Ile Asn Glu Glu Ala
       50                  55                  60
   Ser Glu Trp Asp Arg Thr His Pro Gln Gln Ala Gly Pro Leu Pro Pro
   65                  70                  75                  80
   Gly Gln Ile Arg Asp Pro Thr Gly Ser Asp Ile Ala Gly Thr Thr Ser
                   85                  90                  95
   Thr Gln Gln Glu Gln Val His Trp Thr Thr Arg Ala Ala Asn Pro Ile
               100                 105                 110
   Pro Val Gly Asp Ile Tyr Arg Lys Trp Ile Val Leu Gly Leu Ile Lys
           115                 120                 125
   Met Val Lys Met Tyr
       130
(2) INFORMATIONS POUR LA SEQ ID NO: 74:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "primer"
   (xi) DESCRIPTION DE LA SEQUENCE: SEQ ID NO: 74:
   CAGGGACAAA TGGTACATCA                                              20
(2) INFORMATIONS POUR LA SEQ ID NO: 75:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "primer"
   (xi) DESCRIPTION DE LA SEQUENCE: SEQ ID NO: 75:
   AGTAGCTTGC TCAGCTCTTA AT 22
(2) INFORMATIONS POUR LA SEQ ID NO: 76:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "primer"
   (xi) DESCRIPTION DE LA SEQUENCE: SEQ ID NO: 76:
   TRGTTACTTG TACACATGGC AT 22
(2) INFORMATIONS POUR LA SEQ ID NO: 77:
   (i) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "primer"
   (xi) DESCRIPTION DE LA SEQUENCE: SEQ ID NO: 77:
   ACAATAAAAG AATTCTCCAT GACAGT 26

## Description

La présente invention est relative à des souches de rétrovirus du groupe VIH-1, groupe O et en particulier les souches dénommées BCF02 (ESS), BCF01 (FAN), BCF06 (LOB), BCF07 (MAN), BCF08 (NKO), BCF11 (NAN) et BCF03 (POC), à des fragments desdits rétrovirus ainsi qu'à leurs applications, en tant que réactif de diagnostic et en tant qu'agent immunogène.

Deux types distincts de VIH (virus de l'immunodéficience humaine : VIH-1 et VIH-2) ont été décrits et sont les agents responsables du SIDA. L'analyse de leur séquence d'acide nucléique a permis d'identifier différents sous-types de VIH-1, bien qu'aucune corrélation entre la variabilité et la pathogénicité n'ait pu être établie. De manière similaire, le VIH-2 présente une plus grande diversité génétique et biologique que celle antérieurement envisagée.

L'analyse de fragments nucléotidiques de différents isolats de VIH-1, a montré l'existence, à travers l'analyse du gène env, d'au moins 7 sous-types différents, dénommés A à G (MYERS G. et al., Human retro-viruses and AIDS, 1993, Los Alamos Nat. Lab.).

Plus récemment, deux autres isolats, considérés comme nettement plus distants des 7 autres sous-types, c'est-à-dire dont l'homologie de séquence est la plus distante de celle des souches de références VIH-1, ont également été isolés : VIH-1ANT70 et VIH-1MVP5180, obtenus à partir de patients camerounais, et ont été rattachés à un nouveau groupe de VIH-1, le groupe O, par opposition au groupe M correspondant aux 7 sous-types A-G précités, compte-tenu de leur organisation génomique (5' LTR Gag Pol Vif Vpu Vpr Tat Rev Env Nef LTR 3'), (Demande de Brevet WO 89/12094, Demande de Brevet européen n° 0 591 914, GÛRTLER L.G. et al., J. Virol., 1994, **68**, 1581-85).

L'analyse des séquences d'ADN a montré 65-70 % de similarité avec VIH-1 et 56 % avec VIH-2.

Chameau et al. (Virology 1994, 205, p. 247-253) décrivent l'isolement de la souche VAU du VIH-1 de type O et la comparent aux souches ANT70 et MVP5180. Nkengasong et al. (AIDS 1994, 8, p. 1405-1412) décrivent l'isolement d'une autre souche classée dans le type O sur la base d'une séquence partielle du gène *pol*.

En mettant en oeuvre une méthode d'*immunoblotting* par compétition, utilisant un peptide V3 de MVP5180, une prévalence de 7-8 % est retrouvée à Yaoundé. Cette prévalence pourrait être sous-estimée, dans la mesure où la boucle V3 est connue, dans tous les sous-types d'HIV-1, pour être une région hautement variable. Des études moléculaires indiquent également la présence de virus de groupe O, au Gabon, en France, en Espagne et en Allemagne.

Des études sérologiques en cours mettent en évidence des infections par les VIH-1 du groupe O au Nigeria, au Niger et au Sénégal.

La connaissance de ces différents groupes et sous-types est particulièrement importante pour mettre au point :
- des réactifs de dépistage des infections par VIH, suffisamment sensibles et spécifiques, c'est-à-dire ne conduisant pas à des résultats faussement négatifs ou faussement positifs ; et
- des compositions protectrices vis-à-vis de tous les sous-types existants, y compris l'ensemble des virus du groupe O.

En effet, il a été montré, en particulier, que certains réactifs de détection n'étaient pas suffisamment sensibles et ne permettaient pas toujours de détecter les infections par VIH-1, groupe O (LOUSSERT-AJAKA I. et al., Lancet, 1994, **343**, 1393-94), ce qui a conduit au retrait de trois kits de dépistage et au déclassement de deux autres, sur le marché français.

Les résultats révèlent que les virus de groupe O sont très distants des sous-types du groupe M de VIH-1. Ces résultats indiquent que les virus VIH-1 devraient être classés dans deux groupes différents, à savoir : VIH-1 M et VIH-1 O. Les virus du groupe O semblent pouvoir être transmis par des voies horizontales et verticales, conduisant à une distribution très large de cette infection. La pathogénicité de ces virus est en cours d'étude. La divergence de ces virus doit être prise en compte dans la sensibilité des tests de diagnostic et dans le développement de vaccins.

En conséquence, la Demanderesse s'est donné pour but de pourvoir à des fragments, issus de souches sélectionnées de VIH-1 du groupe O, aptes à permettre à la fois une détection de l'ensemble des virus VIH-1 de groupe O et une différenciation spécifique intra-groupe O et également susceptibles d'induire une protection vis-à-vis de l'ensemble des sous-types de VIH-1, y compris le groupe O, lesquels fragments permettent d'éviter l'obtention de résultats faussement négatifs ou faussement positifs, pour ce faire, les Inventeurs ont sélectionné un ensemble de souches et de séquences, issues essentiellement d'une région du gène env, notamment au niveau d'un fragment hypervariable situé dans la boucle V3 (C2V3) ou au niveau de la gp41 et d'une région du gène *gag*.

La présente invention a pour objet des souches de VIH-1 de groupe O, présentant les caractéristiques morphologiques et immunologiques de l'un des rétrovirus déposés à la Collection Nationale de Cultures de Microorganismes tenue par l'Institut Pasteur, sous les numéros I-1544 (dénommé BCF02 (ESS)), I-1543 (dénommé BCF01 (FAN)), I-1546 (dénommé BCF07 (MAN)), I-1547 (dénommé BCF08 (NKO)), I-1545 (dénommé BCF03 (POC)), en date du 24 février 1995.

La présente invention a également pour objet un fragment d'acide nucléique, caractérisé en ce que sa séquence nucléotidique est choisie parmi celles qui sont contenues dans l'une des séquences nucléotidiques comprises dans les gènes *env* ou *gag* des souches de VIH-1 de groupe O et ses variants et comprend :
- soit l'une des séquences suivantes, incluse dans le fragment de gène C2V3-*env* (boucle hypervariable de la gp120) : SEQ ID N°1 (BCF02 (ESS)), SEQ ID N°2 (BCF08 (NKO)), SEQ ID N°3 (BCF03 (POC)), SEQ ID N°4 (BCF06 (LOB)), SEQ ID N°5 (BCF07 (MAN)), SEQ ID N°6 (BCF01 (FAN)), SEQ ID N°7 (BCF11 (NAN)), SEQ ID N°50 (BCF09), SEQ ID N°51 (BCF12), SEQ ID N°52 (BCF13), SEQ ID N°53 (BCF14),
- soit l'une des séquences suivantes, incluse dans le fragment gp41 du gène *env. :* SEQ ID N°8 (BCF02 (ESS)), SEQ ID N°9 (BCF08 (NKO)), SEQ ID N°10 (BCF03 (POC)), SEQ ID N°11 (BCF06 (LOB)), SEQ ID N°12 (BCF07 (MAN)), SEQ ID N°13 (BCF01 (FAN)), SEQ ID N°14 (BCF11 (NAN)) SEQ ID N°54 (BCF09), SEQ ID N°55 (BCF12), SEQ ID N°56 (BCF13), SEQ ID N°57 (BCF14),
- soit l'une des séquences suivantes, incluse dans le gène *gag :* SEQ ID N°15 (BCF02 (ESS)), SEQ ID N°16 (BCF08 (NKO)), SEQ ID N°17 (BCF03 (POC)), SEQ ID N°18 (BCF06 (LOB)), SEQ ID N°19 (BCF07 (MAN)), SEQ ID N°20 (BCF01 (FAN)), SEQ ID N°21 (BCF11 (NAN)), SEQ ID N°58 (BCF09), SEQ ID N°59 (BCF12), SEQ ID N°60 (BCF13), SEQ ID N°61 (BCF14),
- soit, si la séquence n'est pas identique à l'une des séquences nucléotidiques ci-dessus, ou n'est pas complémentaire de l'une de ces séquences, est néanmoins susceptible de s'hybrider avec une séquence nucléique issue d'un virus VIH-1 de groupe O.

Au sens de la présente invention, on entend par séquence nucléique, les séquences, telles que précisées ci-dessus et leurs séquences complémentaires, ainsi que les séquences les contenant.

De telles séquences trouvent application à la fois dans l'identification spécifique d'un VIH-1 de groupe O, comme réactif de diagnostic, seules ou en pool avec d'autres réactifs, pour l'identification de n'importe quel VIH-1 ou bien, selon les cas, comme réactif de différenciation intra-groupe O.

Ces séquences peuvent notamment être mises en oeuvre dans des tests de diagnostic comprenant, soit une hybridation directe avec la séquence virale à détecter, soit une amplification de ladite séquence virale, en utilisant comme amorces, un oligonucléotide, inclus dans l'une quelconque des séquences ci-dessus et notamment l'une des séquences suivantes :
* séquences *gag*
   GAG/5'CAM ou G5 : CAGGGACAAATGGTACATCA (positions 1250-1269) (SEQ ID N°74)
   GAG/3'CAM ou G3 : AGTAGCTTGCTCAGCTCTTAAT (positions 1768-1747) (SEQ ID N°75)
* séquences gp41
   - SEQ ID N°22 (gp41/5'CAM-1) : AGRGAAAAAAGAGCAGTAGGAT (positions 7800-7821)
   - SEQ ID N°23 (gp41/5'CAM-2) : TCTAAGTGCAGCAGGTAGCACTAT (positions 7843-7866)
   - SEQ ID N°24 (gp41/3'CAM-2) : CTAAGTTGCTCAAGAGTGGTA (positions 8594-8573)
   - SEQ ID N°25 (gp41/3'CAM-1) : GTTGCTCAAGAGGTGGTAAGT (positions 8590-8570)
* séquences C2V3 :
   C2V3/5'CAM ou V3L5 : TRGTTACTTGTACACATGGCAT (positions 6991-7012) (SEQ ID N°76)
   C2V3/3'CAM ou V3L3 : ACAATAAAAGAATTCTCCATGACAGT (positions 7421-7396) (SEQ ID N°77).

Les positions précitées correspondent à celles de la séquence Ant70 (Myers, Korber et al., précité).

La présente invention a également pour objet des souches de VIH-1 de groupe O, caractérisées en ce qu'elles comprennent au moins l'une des séquences sélectionnées dans le groupe constitué par les séquences SEQ ID N°1 à SEQ ID N°7 ou SEQ ID N°50 à SEQ ID N°53, au moins l'une des séquences sélectionnées dans le groupe constitué par les séquences SEQ ID N°8 à SEQ ID N°14 ou SEQ ID N°54 à SEQ ID N°57 et au moins l'une des séquences sélectionnées dans le groupe constitué par les séquences SEQ ID N°15 à SEQ ID N°21 ou SEQ ID N°58 à SEQ ID N°61.

Selon un mode de réalisation avantageux de ladite souche, elle comprend les séquences SEQ ID N°50, SEQ ID N°54, SEQ ID N°58 ; cette souche a été dénommée BCF09.

Selon un autre mode de réalisation avantageux de ladite souche, elle comprend les séquences SEQ ID N°51, SEQ ID N°55, SEQ ID N°59 ; cette souche a été dénommée BCF12.

Selon encore un autre mode de réalisation avantageux de ladite souche, elle comprend les séquences SEQ ID N°52, SEQ ID N°56, SEQ ID N°60 ; cette souche a été dénommée BCF13.

Selon encore un autre mode de réalisation avantageux de ladite souche, elle comprend les séquences SEQ ID N°53, SEQ ID N°57, SEQ ID N°61 ; cette souche a été dénommée BCF14.

Selon un autre mode de réalisation avantageux de ladite souche, elle comprend les séquences SEQ ID N°7, SEQ ID N°14, SEQ ID N°21 ; cette souche a été dénommée BCF11.

Selon encore un autre mode de réalisation avantageux de ladite souche, elle comprend les séquences SEQ ID N°4, SEQ ID N°11, SEQ ID N°18 ; cette souche a été dénommée BCF06.

L'invention a également pour objet l'utilisation des séquences décrites ci-dessus pour la mise en oeuvre d'un procédé d'hybridation ou d'amplification génique de séquences nucléiques du type VIH-1, ces procédés étant applicables au diagnostic *in vitro* de l'infection potentielle d'un individu par un virus du type VIH-1, y compris le groupe O.

Cette méthode de diagnostic *in vitro* est réalisée à partir d'un échantillon biologique (sérum, lymphocytes circulants) et comprend :
. une étape d'extraction de l'acide nucléique à détecter, appartenant au génome du virus du type VIH-1, éventuellement présent dans l'échantillon biologique et le cas échéant une étape de traitement de l'acide nucléique, à l'aide d'une trancriptase inverse, si ce dernier est sous forme d'ARN,
. au moins un cycle comprenant les étapes de dénaturation de l'acide nucléique, d'hybridation avec au moins une séquence conforme à l'invention et extension de l'hybride formé, en présence des réactifs convenables (agent de polymérisation, tel qu'ADN polymérase et dNTP) et
. une étape de détection de la présence éventuelle de l'acide nucléique appartenant au génome d'un virus de type VIH-1 de groupe O (spécificité de groupe).

L'invention a également pour objet un peptide, caractérisé en ce qu'il est exprimé par une séquence nucléotidique telle que définie ci-dessus.

Parmi ces peptides, on peut citer, en particulier :
- ceux exprimés par le fragment de gène C2V3-*env*, conforme à l'invention : SEQ ID N°26 (BCF02 (ESS)), SEQ ID N°27 (BCF01 (FAN)), SEQ ID N°28 (BCF01 (FAN)), SEQ ID N° 29 (BCF06 (LOB)), SEQ ID N°30 (BCF07 (MAN)), SEQ ID N°31 (BCF11 (NAN)), SEQ ID N°32 (BCF08 (NKO)), SEQ ID N°33 (BCF08 (NKO)), SEQ ID N°34 (BCF03 (POC)), SEQ ID N°35 (BCF03 (POC)), SEQ ID N°62 (BCF09), SEQ ID N°63 (BCF12), SEQ ID N°64 (BCF13), SEQ ID N°65 (BCF14),
- ceux exprimés par le fragment de gène gp41 env, conforme à l'invention : SEQ ID N°36 (BCF02 (ESS)), SEQ ID N°37 (BCF01 (FAN)), SEQ ID N°38 (BCF06 (LOB)), SEQ ID N°39 (BCF07 (MAN)), SEQ ID N°40 (BCF08 (NKO)), SEQ ID N°41 (BCF03 (POC)), SEQ ID N°42 (BCF11 (NAN)), SEQ ID N°66 (BCF09), SEQ ID N°67 (BCF12), SEQ ID N°68 (BCF13), SEQ ID N°69 (BCF14),
- ceux exprimés par le fragment de gène *gag* conforme à l'invention : SEQ ID N°43 (BCF02 (ESS)), SEQ ID N°44 (BCF01 (FAN)), SEQ ID N°45 (BCF06 (LOB)), SEQ ID N°46 (BCF07 (MAN)), SEQ ID N°47 (BCF11 (NAN)), SEQ ID N°48 (BCF08 (NKO)), SEQ ID N°49 (BCF03 (POC)), SEQ ID N°70 (BCF09), SEQ ID N°71 (BCF12), SEQ ID N°72 (BCF13), SEQ ID N°73 (BCF14).

L'invention a également pour objet des compositions immunogènes comprenant un ou plusieurs produits de traduction des séquences nucléotidiques selon l'invention ou un fragment de ceux-ci et/ou au moins l'un des peptides tels que définis ci-dessus.

L'invention a également pour objet les anticorps dirigés contre l'un ou plusieurs des peptides décrits ci-dessus et leur utilisation pour la mise en oeuvre de méthodes de diagnostic *in vitro* de l'infection d'un individu par un virus de type VIH-1, selon les procédés connus de l'Homme du métier.

A titre d'illustration, une telle méthode de diagnostic *in vitro* selon l'invention comprend la mise en contact d'un échantillon biologique prélevé chez un patient, avec des anticorps selon l'invention, et la détection à l'aide de tout procédé approprié, notamment à l'aide d'anti-immunoglobulines marquées, des complexes immunologiques formés entre les antigènes des virus du type VIH-1 éventuellement présents dans l'échantillon biologique et lesdits anticorps.

La présente invention a également pour objet un procédé de criblage et de typage de VIH-1, groupe O, caractérisé en ce qu'il comprend la mise en contact de l'un quelconque des fragments nucléotidiques conformes à l'invention avec l'acide nucléique du virus à typer et la détection de l'hybride formé.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

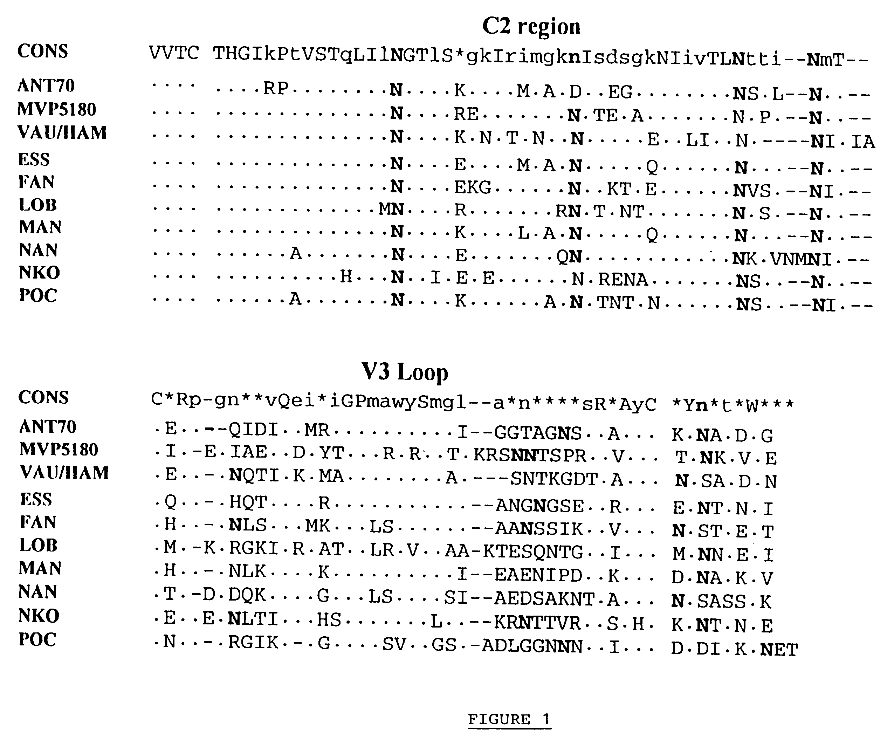

- la figure 1 représente les séquences comparées d'aminoacides exprimés par le fragment de gène env C2V3;

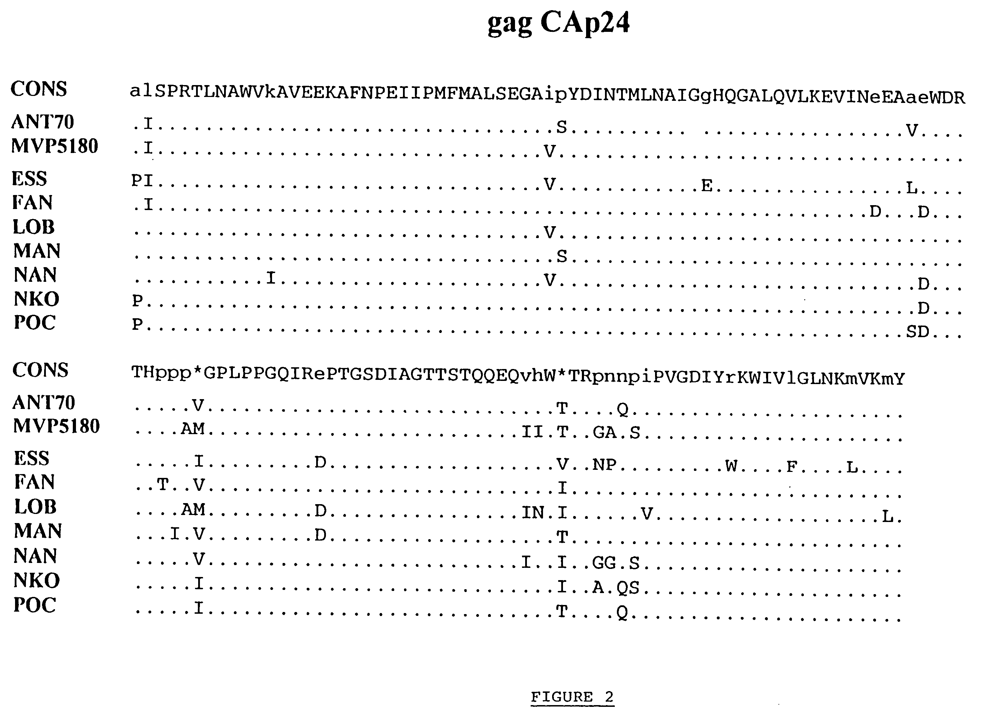

- la figure 2 représente les séquences comparées d'aminoacides exprimés par le fragment de gène *gag* ;

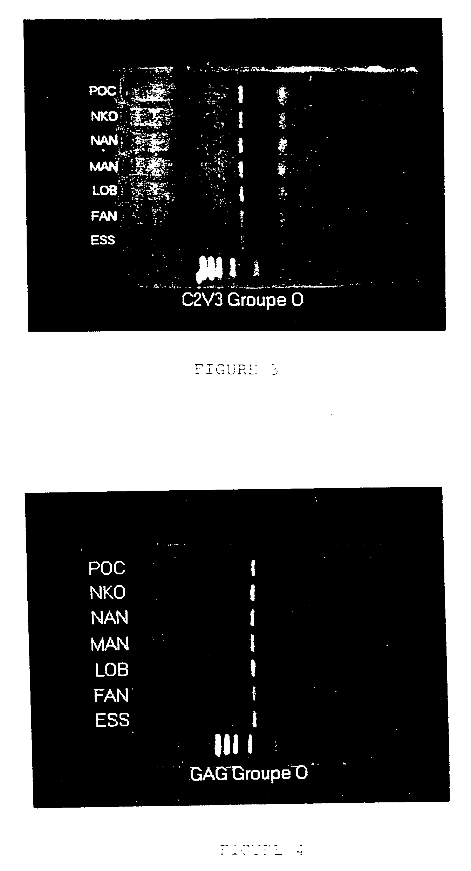

- la figure 3 illustre les résultats obtenus avec les 7 souches BCF02 (ESS), BCF01 (FAN), BCF07 (MAN), BCF11 (NAN), BCF08 (NKO), BCF03 (POC) et BCF06 (LOB) sur gel d'agarose, d'une PCR réalisée avec les amorces C2V3 précitées ;

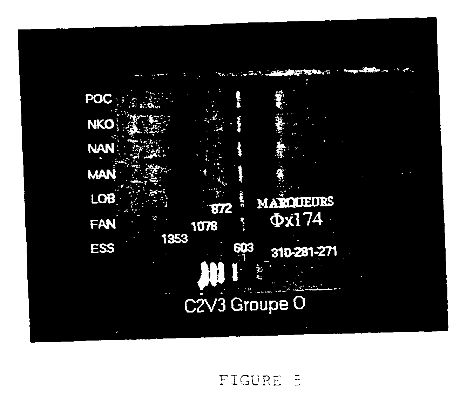

- la figure 4 illustre les résultats obtenus avec les 7 souches BCF02 (ESS), BCF01 (FAN), BCF07 (MAN), BCF11 (NAN), BCF08 (NKO), BCF03 (POC) et BCF06 (LOB) sur gel d'agarose, d'une PCR réalisée avec les amorces Gag précitées ;
- la figure 5 représente les marqueurs utilisés dans les figures 3 et 4 ;
- la figure 6 illustre la réactivité des sérums correspondant aux souches selon l'invention, par rapport à l'organisation phénétique de ces variants ;
- la figure 7 illustre une analyse phylogénétique basée sur la région gag.

Il doit être bien entendu, toutefois, que ces exemples sont donnés uniquement à titre d'illustration de l'objet de l'invention, dont ils ne constituent en aucune manière une limitation.

### EXEMPLE 1 : Obtention des séquences conformes à l'invention.

### Patients :

Sept patients camerounais, consultants ou hospitalisés dans les hôpitaux de Paris sont inclus dans cette étude.

Au moment du diagnostic de la séropositivité, quatre patients sont au stade de SIDA (CDC IVA n=1, CDC IVC1 n=3) et 3 patients sont asymptomatiques (CDC II). La tranche d'âge varie entre 22 à 44 ans pour les patients symptomatiques et elle s'étale entre 22 et 68 ans pour les patients asymptomatiques.

Quatre patients ont un taux de CD4+ < 200x10⁶/µl (10, 19, 91, 97), 2 patients ont des CD4+ compris entre 200 et 500x10⁶/µl (384, 420) et un patient a des CD4+ >500x10⁶/µl (575).

### Cultures :

Dix à vingt ml de sang total pour ces 7 patients ont été recueillis sur héparine de lithium. Les cellules mononucléées du sang périphérique (CMSP) sont isolées sur un gradient de centrifugation Ficoll-Hypaque (Pharmacia). Le culot cellulaire obtenu est lavé 2 fois avec du RPMI 1640 et resuspendu dans du milieu de culture à une concentration de 2.10⁶ cellules/ml. Ce milieu de culture contient du RPMI 1640 complété avec 20 % du sérum de veau foetal, 10 % d'interleukine humaine, 150 µg/ml de streptomycine, 250 unités /ml de pénicilline G et 5 % de L-glutamine. Un million des cellules du patient est co-cultivé en duplicate avec un million de cellules de donneurs stimulés au PHA dans des plaques de culture 24 puits (Costar). Le milieu de culture est changé 2 fois par semaine, et 3x10⁵ cellules de donneurs sont rajoutées à chaque puits à j7, j14 et j21.

La réplication virale est surveillée dans les surnageants de culture pendant 28 jours, simultanément par une microtechnique (mesure de l'activité transriptase réverse) et la détection de l'antigène p24 (ELAVIA® p24 Ag, Sanofi-Diagnostic Pasteur). Les surnageants de cultures positifs sont recueillis et garder à -80°C pour les réinoculations.

### Préparation de l'ADN :

Une PCR est réalisée à partir de l'ADN de lymphocytes frais extraits d'un patient ou de lymphocytes, après 6 jours de coculture ou du plasma après RT. A partir de 100 µl d'un mélange réactionnel, contenant du Tris-HCl 10 mmol/l pH 8,3, du KCl 50 mmol/l, du MgCl₂ 2 mmol/l, 0,2 mmol/l de chaque dNTP, 40 pmole de chaque amorce, 2,5 U de *Taq* polymérase (Perkin Elmer Cetus, St Quentin Yvelines, France) et 1 µg d'ADN cellulaire. Les amorces utilisées sont celles précisées ci-dessus, à savoir :
* séquences gag :
   GAG/5'CAM : CAGGGACAAATGGTACATCA (positions 1250-1269) (SEQ ID N°74) ; GAG/3'CAM : AGTAGCTTGCTCAGCTCTTAAT (positions 1768-1747) (SEQ ID N°75)
* séquences gp41
   gp41/5'CAM-1 : AGRGAAAAAAGAGCAGTAGGAT (positions 7800-7821) (SEQ ID N°22)
   gp41/5'CAM-2 : TCTAAGTGCAGCAGGTAGCACTAT (positions 7843-7866) (SEQ ID N°23)
   gp41/3'CAM-2 : CTAAGTTGCTCAAGAGTGGTA (positions 8594-8573) (SEQ ID N°24)
   gp41/3'CAM-1 : GTTGCTCAAGAGGTGGTAAGT (positions 8590-8570) (SEQ ID N°25)
* ou bien l'une des séquences suivantes : SEQ ID N°76 et SEQ ID N°77 pour la région *env* et SEQ ID N°74 et SEQ ID N°75 pour la région *gag*, correspondant respectivement aux nucléotides 6991-7012, 7421-7396 et 1250-1269, 1768-1747 de la séquence HIV1^{Ant70}.

Les échantillons sont soumis à 40 cycles d'amplification, chaque cycle comprenant les trois étapes suivantes : dénaturation à 94°C pendant une minute, hybridation des amorces à 50°C pour la séquence *gag* et à 55°C pour la séquence *env,* pendant une minute et extension à 72°C pendant une minute. Lors du premier cycle, la dénaturation est réalisée pendant 4 minutes et pour le dernier cycle, l'extension est réalisée pendant 5 minutes. Les produits amplifiés sont soumis à une digestion enzymatique (*Xho1, EcoR1),* purifiés et clonés dans un vecteur M13mp18, digéré par les enzymes de restriction *Sal1* and *EcoR1*.

Pour chaque patient, entre 3 et 4 clones sont séquencés (Applied 373A sequencer), dans une région de 406 pb du gène *gag*, dans une région de 320 pb du gène *env* comprise dans la région V3 et au niveau de la région codant pour la gp41.

### EXEMPLE 2 : Immunodétection d'un VIH-1 de groupe O.

Des tests ELISA sont réalisés dans des plaques de microtitration (Falcon 3912, microtest III®, Becton Dickinson).

Les puits sont recouverts de 100 µl de l'un des peptides V3 définis ci-dessus : BCF08 (NKO) : R T I Q E I H S G P M A W Y S L G L K R N T T V R (SEQ ID N°33) ; BCF01 (FAN) : R S V Q E M K I G P L S W Y S M G L A A N S S I K (SEQ ID N°28) ; BCF03 (POC) : R I K Q I G I G P M S V Y S G S L A D L G N N N (SEQ ID N°35), dilué à 10 µg.ml⁻¹ dans un tampon carbonate 0,05 M, pH 9,6 par incubation une nuit, à +4°C.

Les plaques sont ensuite lavées trois fois avec un tampon PBS contenant du Tween® 20 (Prolabo, France),(PBS-Tween®) 0,1 %, puis saturées avec du PBS, supplémenté avec du lait à 1 %, (Gloria Co., France), pendant 1h à 37°C.

Des sérums (1/100), dilués dans un mélange PBS-lait contenant du Tween® 20 à 0,1 % (PBS-lait-Tween®), sont incubés pendant 2h à 37°C.

Après trois lavages, des anticorps polyvalents anti-humains, conjugués à de la phosphatase alcaline (Sigma), dilués au 1:10 000 dans du PBS-lait-Tween, sont ajoutés et incubés pendant 1h à 37°C. Après le dernier lavage, la réaction colorée est développée à 37°C, avec un substrat de la phosphatase alcaline, le p-nitrophenyl phosphate (Sigma), dilué dans un tampon carbonate à 0,05 M, pH 9,5, contenant du MgCl₂ 2 mM, pour obtenir une concentration de 1 mg.ml⁻¹. L'absorbance, mesurée à 405 nm (A₄₀₅) est enregistrée avec un appareil (MR 5000, Dynatech). Un seuil de coupure est déterminé pour chaque sérum testé et correspond à trois fois la valeur A₄₀₅ obtenue avec le peptide E19S, dérivé de *Plasmodium malariae.*

Le Tableau I ci-après montre les résultats obtenus.

Les séquences consensus et FR 15-1 correspondent à celles obtenues à partir du sous-type B, retrouvé en France.

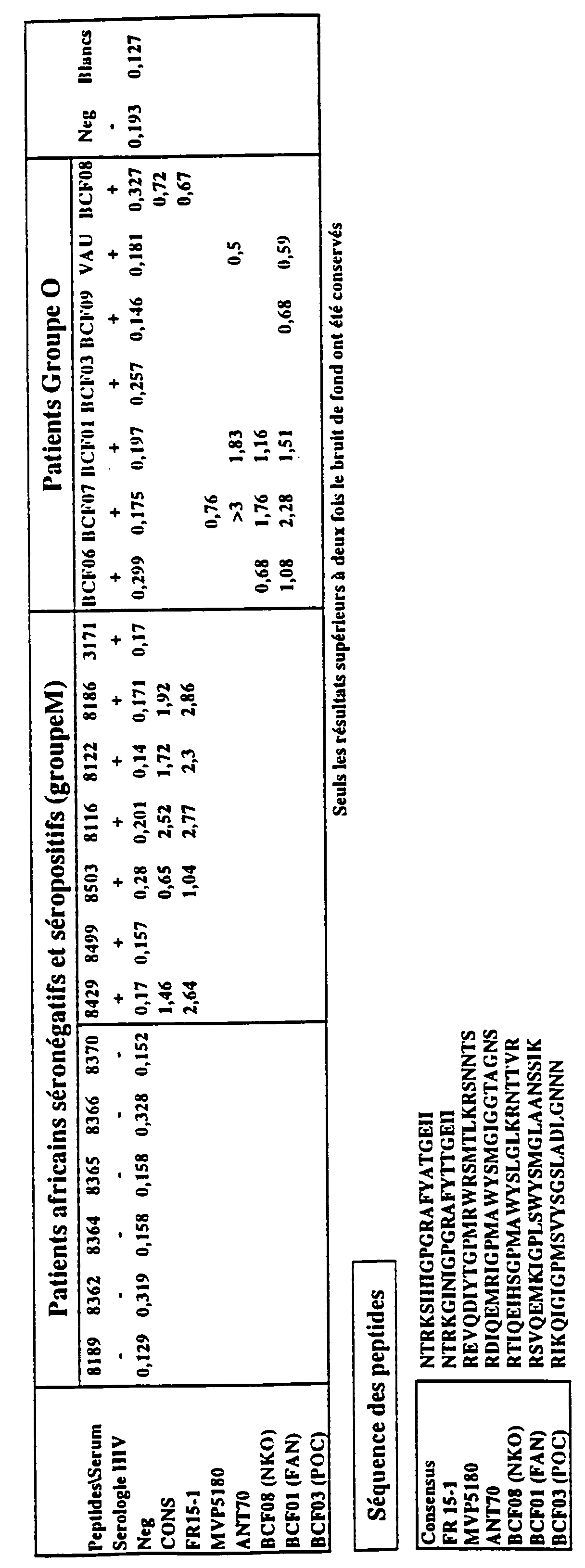

Ce Tableau I montre la spécificité des peptides selon l'invention.

### EXEMPLE 3 : Particularités sérotypiques, phénotypiques et génotypiques des souches selon l'invention.

Ces particularités sont illustrées aux Tableaux II et III ci-après.

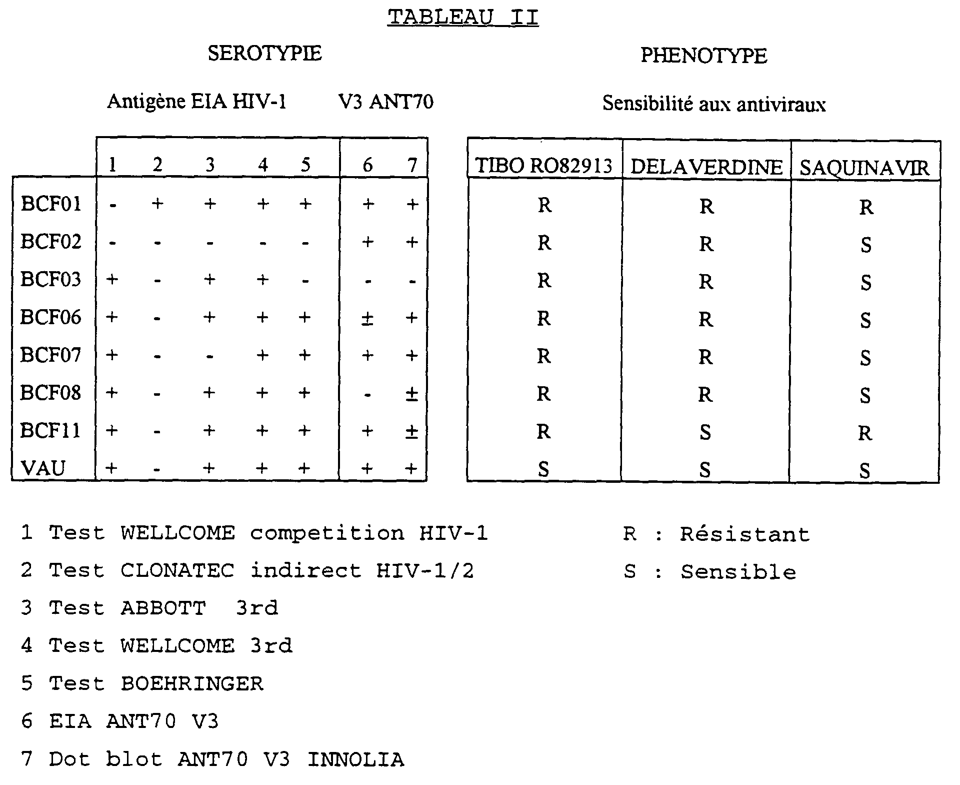

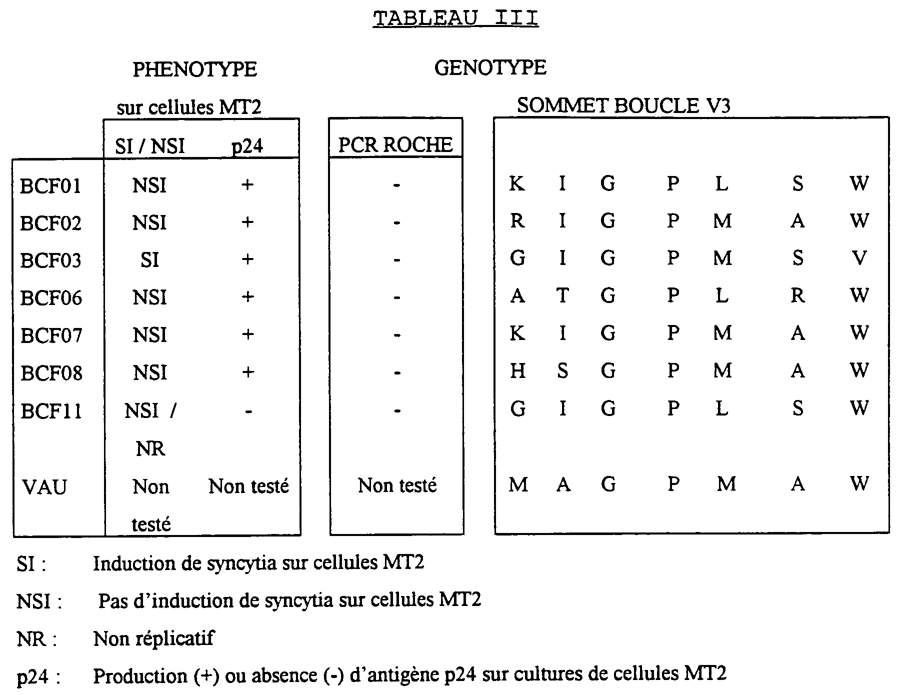

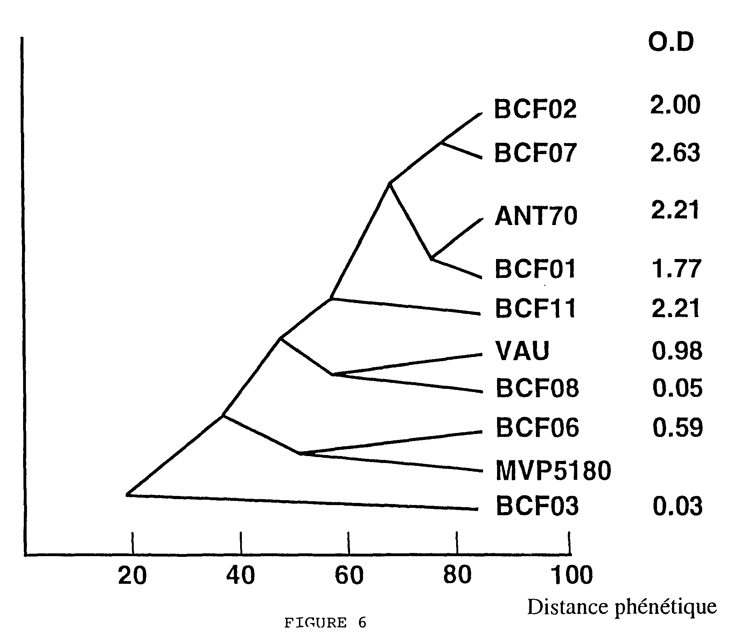

L'analyse sérotypique sur un ensemble de tests commercialisés (Tableau II) (à l'exception du test n° 6, EIA Ag V3 ANT70, produit de recherche non commercialisé) met en évidence la grande diversité de réponse anticorps vis-à-vis des antigènes du sous-type B dominant en occident et par rapport à l'antigène de la boucle V3 de la souche ANT70. Les sérums, correspondant à chacun des isolats selon l'invention, présentent un profil unique et caractéristique. BCF 01 est le seul positif sur le test 2 (Clonatec®). BCF 02 est complétement négatif sur les antigènes sous-type B. BCF 03, inversement, réagit sur le sous-type B mais est négatif sur les antigènes ANT70. BCF 07 est le seul négatif sur les tests n° 2 (Clonatec) et n° 3 (Abbott 3rd), mais réactif sur l'ensemble des autres tests avec antigène du groupe M. BCF 08 est strictement négatif sur le test EIA V3 ANT70 mais comme BCF 11, faiblement réactif sur cet antigène par le test 7 (InnoLIA). Par comparaison, le sérum correspondant à la souche VAU, qui a été caractérisée moléculairement par CHARNEAU et al. (Virology, 1994, **205**, 247-253), est positif sur tous ces antigènes à l'exception du test 2 ((Clonatec®). Cette diversité de réponse anticorps est à interpréter comme le reflet de la diversité antigénique de ces souches. Un lien avec l'immunodépression est exclu, les patients BCF 07, 08 et 11 étant complètement asymptomatiques avec un nombre de CD4 >400/ml. La figure 6 indique la réactivité des sérums correspondant aux souches selon l'invention par rapport à l'organisation phénétique de ces variants.

La caractérisation phénotypique des isolats selon l'invention montre l'importance et la nécessité de disposer d'un grand nombre de réactifs sensibles. Tous sont résistants naturellement à la molécule Tibo Ro82913 (Tableau III) comme déjà rapporté pour VIH-2. Une absence d'activité inhibitrice de croissance *in vitro* en dehors d'un traitement par Ro82913 n'a jamais été rapportée pour VIH-1 auparavant. En contraste, la souche VAU est parfaitement sensible au Ro82913.

Les souches sont également résistantes a un autre inhibiteur non nucléosidique, la Delaverdine, à l'exception de la souche BCF 11, qui est sensible.

Inversement, cette souche est résistante au Saquinavir, une anti-protéase, alors que les autres souches y sont sensibles.

Cette diversité de réponse aux anti-rétro-viraux renforce la notion d'une grande dispersion au sein même des variants du Cameroun.

De plus, les résultats de formation de syncytia sur la lignée continue MT2 complique toute classification puisque sans corrélation aux résultats sérotypiques ou génotypiques précédents et sans rapport au stade clinique. Ces souches n'induisent pas de formation syncytiale alors que la détection d'antigène p24 dans les surnageants confirme la réplication sur cette lignée.

Enfin, au niveau génotypique, toutes les amplifications des génomes des souches selon l'invention sont négatives par PCR utilisant un kit commercialisé par Roche Diagnostics, qui utilise des amorces et des sondes correspondant à une région conservée du gène Gag de VIH-1 ; une négativité de ce test était également possible dans les sous-types A de VIH-1 (LOUSSERT-AJAKA et al., 1995, Lancet, **346**, 912-913 ; LOUSSERT-AJAKA et al., 1995, **346**, 1489). Cette négativité au sein des isolats variants du Cameroun mais aussi au sein des sous-types A renforce cette notion de grande variabilité VIH-1 et montre la nécessité pour la détection des acides nucléiques VIH-1, d'utiliser un large panel de séquences et en particulier celles selon la présente invention.

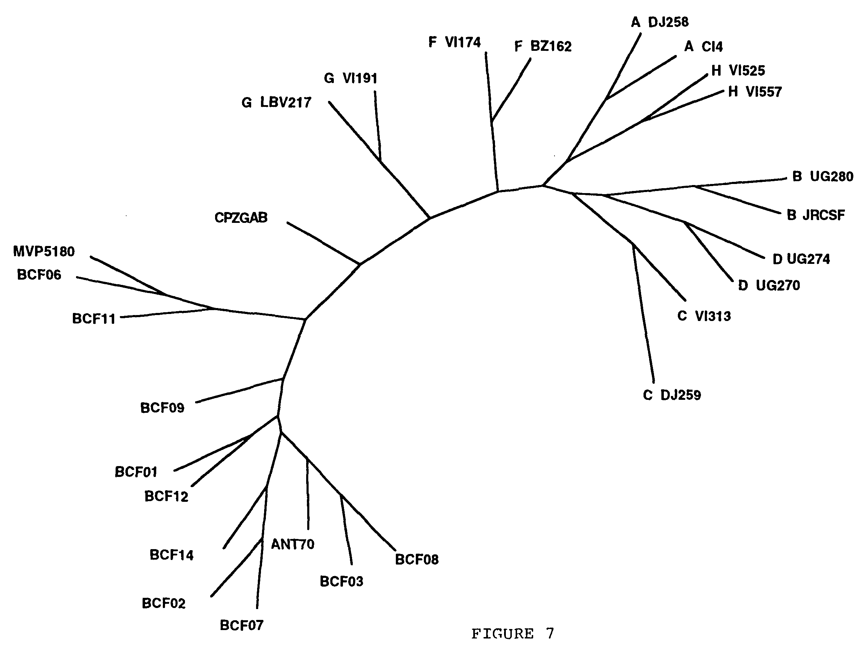

Les figures 6 et 7 correspondent à une analyse phylogénétique basée sur la région de *gag*.

La phylogénie des gènes *gag* confirme la dispersion de ces variants et la difficulté de les regrouper soit en sous-type, soit même en un groupe d'exclusion et montre la nécessité d'une sélection de variants présentant des caractères phénétiques et génétiques particuliers et dont les sérums correspondant ont des profils de réactivité ou de non-réactivité caractéristiques, aptes à permettre la détection de VIH-1 dans des sérums précédemment considérés comme faussement négatifs.

## Revendications

1. Souches de VIH-1 de groupe O, **caractérisées en ce qu'**elles comprennent au moins l'une des séquences sélectionnées dans le groupe constitué par les séquences SEQ ID N°1 à SEQ ID N°7 ou SEQ ID N°50 à SEQ ID N°53, au moins l'une des séquences sélectionnées dans le groupe constitué par les séquences SEQ ID N°8 à SEQ ID N°14 ou SEQ ID N°54 à SEQ ID N°57 et au moins l'une des séquences sélectionnées dans le groupe constitué par les séquences SEQ ID N°15 à SEQ ID N°21 ou SEQ ID N°58 à SEQ ID N°61.

2. Souches de VIH-1 de groupe O selon la revendication 1, **caractérisées en ce qu'**elles sont sélectionnées dans le groupe constitué par :
- la souche qui comprend les séquences SEQ ID N°1, SEQ ID N°8 et SEQ ID N°15, dénommée BCF02 ;
- la souche qui comprend les séquences SEQ ID N°2, SEQ ID N°9 et SEQ ID N°16, dénommée BCF08 ;
- la souche qui comprend les séquences SEQ ID N°3, SEQ ID N°10 et SEQ ID N°17, dénommée BCF03 ;
- la souche qui comprend les séquences SEQ ID N°4, SEQ ID N°11 et SEQ ID N°18, dénommée BCF06 ;
- la souche qui comprend les séquences SEQ ID N°5, SEQ ID N°12 et SEQ ID N°19, dénommée BCF07 ;
- la souche qui comprend les séquences SEQ ID N°6, SEQ ID N°13 et SEQ ID N°20, dénommée BCF01 ;
- la souche qui comprend les séquences SEQ ID N°7, SEQ ID N°14 et SEQ ID N°21, dénommée BCF11 ;
- la souche qui comprend les séquences SEQ ID N°50, SEQ ID N°54, SEQ ID N°58, dénommée BCF09 ;
- la souche qui comprend les séquences SEQ ID N°51, SEQ ID N°55, SEQ ID N°59, dénommée BCF12 ;
- la souche qui comprend les séquences SEQ ID N°52, SEQ ID N°56, SEQ ID N°60, dénommée BCF13 ;
- la souche qui comprend les séquences SEQ ID N°53, SEQ ID N°57, SEQ ID N°61, dénommée BCF14.

3. Souches de VIH-1 de groupe O selon la revendication 2, déposées à la Collection Nationale de Cultures de Microorganismes tenue par l'Institut Pasteur, sous les numéros I-1544 (dénommé BCF02 (ESS)), I-1543 (dénommé BCF01 (FAN)), I-1546 (dénommé BCF07 (MAN)), I-1547 (dénommé BCF08 (NKO)), I-1545 (dénommé BCF03 (POC)), en date du 24 février 1995.

4. Fragment d'acide nucléique issu d'un VIH-1 de groupe O, **caractérisé en ce que** sa séquence nucléotidique est choisie parmi celles qui sont contenues dans l'une des séquences nucléotidiques comprises dans les gènes *env* ou *gag* et comprend :
- soit l'une des séquences SEQ ID N°1, SEQ ID N°2, SEQ ID N°3, SEQ ID N°4, SEQ ID N°5, SEQ ID N°6, SEQ ID N°7, SEQ ID N°50, SEQ ID N°51, SEQ ID N°52, SEQ ID N°53, incluse dans le fragment de gène C2V3-*env* (boucle hypervariable de la gp120) ;
- soit l'une des séquences SEQ ID N°8, SEQ ID N°9, SEQ ID N°10, SEQ ID N°11, SEQ ID N°12, SEQ ID N°13, SEQ ID N°14, SEQ ID N° 54, SEQ ID N°55, SEQ ID N°56, SEQ ID N°57, incluse dans le fragment gp41 du gène *env*. ;
- soit l'une des séquences SEQ ID N°15, SEQ ID N°16, SEQ ID N°17, SEQ ID N°18, SEQ ID N°19, SEQ ID N°20, SEQ ID N°21, SEQ ID N°58, SEQ ID N°59, SEQ ID N°60, SEQ ID N°61, incluse dans le gène *gag*.

5. Procédé de détection d'un VIH-1 de groupe O par hybridation ou amplification génique, réalisé à partir d'un échantillon biologique (sérum, lymphocytes circulants), lequel procédé est **caractérisé en ce qu'**il comprend :
. une étape d'extraction de l'acide nucléique à détecter, appartenant au génome du virus du type VIH-1, éventuellement présent dans l'échantillon biologique et le cas échéant une étape de traitement de l'acide nucléique, à l'aide d'une trancriptase inverse, si ce dernier est sous forme d'ARN,
. au moins un cycle comprenant les étapes de dénaturation de l'acide nucléique, d'hybridation avec au moins une séquence selon la revendication 4 et extension de l'hybride formé, en présence des réactifs convenables (agent de polymérisation, tel qu'ADN polymérase et dNTP) et
. une étape de détection de la présence éventuelle de l'acide nucléique appartenant au génome d'un virus de type VIH-1 de groupe O.

6. Peptide, **caractérisé en ce qu'**il est exprimé par une souche de VIH-1 selon la revendication 1 ou la revendication 2, ou codé par une séquence nucléotidique selon la revendication 4, et **en ce qu'**il est sélectionné dans le groupe constitué par :
- ceux exprimés par le fragment de gène C2V3-*env*, conforme à l'invention, à savoir : SEQ ID N°26, SEQ ID N°27, SEQ ID N°28, SEQ ID N°29, SEQ ID N°30, SEQ ID N°31, SEQ ID N°32, SEQ ID N°33, SEQ ID N°34, SEQ ID N°35, SEQ ID N°62, SEQ ID N°63, SEQ ID N°64, SEQ ID N°65,
- ceux exprimés par le fragment de gène gp41 *env*, conforme à l'invention, à savoir : SEQ ID N°36, SEQ ID N°37, SEQ ID N°38, SEQ ID N°39, SEQ ID N°40, SEQ ID N°41, SEQ ID N°42, SEQ ID N°66, SEQ ID N°67, SEQ ID N°68, SEQ ID N°69,
- ceux exprimés par le fragment de gène *gag* conforme à l'invention, à savoir : SEQ ID N°43, SEQ ID N°44, SEQ N°45, SEQ ID N°46, SEQ ID N°47, SEQ ID N°48, SEQ ID N°49, SEQ ID N°70, SEQ ID N°71, SEQ ID N°72, SEQ ID N°73.

7. Compositions immunogènes, **caractérisées en ce qu'**elles comprennent un ou plusieurs produits de traduction des séquences nucléotidiques selon la revendication 4 et/ou au moins l'un des peptides selon la revendication 6.

8. Anticorps dirigés contre un ou plusieurs des peptides selon la revendication 6.

9. Méthode de diagnostic *in vitro* d'un VIH-1 de groupe O, **caractérisée en ce qu'**elle comprend la mise en contact d'un échantillon biologique prélevé chez un patient, avec des anticorps selon la revendication 8 et la détection des complexes immunologiques formés entre les antigènes de VIH-1, éventuellement présents dans l'échantillon biologique et lesdits anticorps.

10. Procédé de criblage et de typage de VIH-1, groupe O, **caractérisé en ce qu'**il comprend la mise en contact de l'un quelconque des fragments nucléotidiques selon la revendication 4 avec l'acide nucléique du virus à typer et la détection de l'hybride formé.

11. Réactif de diagnostic d'un VIH-1 de groupe O, **caractérisé en ce qu'**il comprend une séquence selon la revendication 4 ou 6.

12. Réactif de diagnostic, **caractérisé en ce qu'**il est sélectionné dans le groupe constitué par les séquences ID N°22, 23, 24 et 25, lesquelles séquences sont aptes à servir d'amorces pour l'amplification d'un fragment gp41 d'un virus VIH-1 de groupe O.

## Patentansprüche

1. VIH-1 Stämme der Gruppe O, **dadurch gekennzeichnet, daß** sie mindestens eine der Sequenzen ausgewählt aus der Gruppe gebildet aus den Sequenzen SEQ ID N°1 bis SEQ ID N°7 oder SEQ ID N°50 bis SEQ ID N°53, mindestens eine der Sequenzen ausgewählt aus der Gruppe gebildet aus den Sequenzen SEQ ID N°8 bis SEQ ID N°14 oder SEQ ID N°54 bis SEQ ID N°57 und eine der Sequenzen ausgewählt aus der Gruppe gebildet aus den Sequenzen SEQ ID N°15 bis SEQ ID N°21 oder SEQ ID N°58 bis SEQ ID N°61 umfassen.

2. VIH-1 Stämme der Gruppe O gemäß dem Anspruch 1, **dadurch gekennzeichnet, daß** sie ausgewählt sind aus der Gruppe gebildet aus:
- dem Stamm, welcher die Sequenzen SEQ ID N°1, SEQ ID N°8 und SEQ ID N°15 umfaßt, benannt BCF02;
- dem Stamm, welcher die Sequenzen SEQ ID N°2, SEQ ID N°9 und SEQ ID N°16 umfaßt, benannt BCF08;
- dem Stamm, welcher die Sequenzen SEQ ID 20 N°3, SEQ ID N°10 et SEQ ID N°17 umfaßt, benannt BCF03;
- dem Stamm, welcher die Sequenzen SEQ ID N°4, SEQ ID N°11 et SEQ ID N°18 umfaßt, benannt BCF06;
- dem Stamm, welcher die Sequenzen SEQ ID N°5, SEQ ID N°12 et SEQ ID N°19 umfaßt, benannt BCF07;
- dem Stamm, welcher die Sequenzen SEQ ID N°6, SEQ ID N°13 et SEQ ID N°20 umfaßt, benannt BCFO1 ;
- dem Stamm, welcher die Sequenzen SEQ ID N°7, SEQ ID N°14 et SEQ ID N°21 umfaßt, benannt BCF11;
- dem Stamm, welcher die Sequenzen SEQ ID 30 N°50, SEQ ID N°54, SEQ ID N°58 umfaßt, benannt BCF09;
- dem Stamm, welcher die Sequenzen SEQ ID N°51, SEQ ID N°55, SEQ ID N°59 umfaßt, benannt BCF12;
- dem Stamm, welcher die Sequenzen SEQ ID N°52, SEQ ID N°56, SEQ ID N°60 umfaßt, benannt BCF13;
- dem Stamm, welcher die Sequenzen SEQ ID N°53, SEQ ID N°57, SEQ ID N°61 umfaßt, benannt BCF14.

3. VIH-1 Stämme der Gruppe O gemäß dem Anspruch 2, am 29.Februar 1995 hinterlegt bei der Collection Nationale de Cultures de Microorganismes, betrieben durch das Institut Pasteur,unter den Nummern I-1544 (benannt BCF02 (ESS)), 1-1543 (benannt BCFO1 (FAN)), I-1546 (benannt BCF07 (MAN)), I-1547 (benannt BCF08 (NKO)), I-1545 (benannt BCF03 (POC)).

4. Teil einer Nukleinsäure abgeleitet von VIH-1 der Gruppe O, **dadurch gekennzeichnet, daß** dessen Nukleotidsequenz ausgewählt ist aus einer der Nukleotidsequenzen, welche in einem der Gene *env* oder *gag* enthalten sind und umfassen:
- entweder eine der Sequenzen SEQ ID N°1, SEQ ID N°2, SEQ ID N°3, SEQ ID N°4, SEQ ID N°5, SEQ ID N°6, SEQ ID N°7, SEQ ID N°50, SEQ ID N°51, SEQ ID N°52, SEQ ID N°53, enthalten in dem Teil des Gens C2V3-*env* (hypervariable Schleife von gp120) ;
- oder eine der Sequenzen SEQ ID N°8, SEQ ID N°9, SEQ ID N°10, SEQ ID N°11, SEQ ID N°12, SEQ ID N°13, SEQ ID N°14, SEQ ID N° 54, SEQ ID N°55, SEQ ID N°56, SEQ ID N°57, enthalten in dem Teil gp41 des Gens *env*.;
- oder eine der Sequenzen SEQ ID N°15, SEQ ID N°16, SEQ ID N°17, SEQ ID N°18, SEQ ID N°19, SEQ ID N°20, SEQ ID N°21, SEQ ID N°58, SEQ ID N°59, SEQ ID N°60, SEQ ID N°61, enthalten in dem Gen *gag*.

5. Verfahren zur Erfassung eines VIH-1 der Gruppe 0 mittels Bastardisierung oder Genverstärkung, ausgehend von einer biologischen Probe (Serum, zirkulierende Lymphocyten) durchgeführt, welches Verfahren **dadurch gekennzeichnet ist, daß** es umfaßt:
- einen Extraktionsschritt der zu erkennenden Nukleinsäure, welche zu dem Genom des Virus vom Typ VIH-1 gehört, welches möglicherweise in der biologischen Probe vorhanden ist, und gegebenenfalls einen Behandlungsschritt der Nukleinsäure mit Hilfe einer inversen Transkription, falls letztere in Form von ARN vorliegt,
- mindestens einen Zyklus umfassend die Schritte Denaturierung der Nukleinsäure, Bastardisierung mit mindestens einer Sequenz nach dem Anspruch 4 und Erweiterung des gebildeten Bastard, in Anwesenheit von geeigneten Reagenzien (Polymerisationsmittel, wie zum Beispiel ADN Polymerase und dNTP) und
- einen Erfassungsschritt des möglichen Vorhandenseins der Nukleinsäure, welche zu dem Genom eines Virus vom Typ VIH-1 der Gruppe 0 gehört.

6. Peptid,**dadurch gekennzeichnet, daß** es durch einen VIH-1 Stamm nach dem Anspruch 1 oder dem Anspruch 2 ausgedrückt ist oder durch eine Nukleotidsequenz nach Anspruch 4 verschlüsselt ist, und daß es ausgewählt ist aus der Gruppe gebildet aus:
- jenen, welche durch das Teil des Gens C2V3*env* in Einklang mit der Erfindung ausgedrückt sind, das heißt: SEQ ID N°26, SEQ ID N°27, SEQ ID N°28, SEQ ID N°29, SEQ ID N°30, SEQ ID N°31, SEQ ID N°32, SEQ ID N°33, SEQ ID N°34, SEQ ID N°35, SEQ ID N°62, SEQ ID N°63, SEQ ID N°64, SEQ ID N°65,
- jenen, welche durch das Teil des Gens gp41 *env* in Einklang mit der Erfindung gebildet sind, das heißt: SEQ ID N°36, SEQ ID N°37, SEQ ID N°38, SEQ ID N°39, SEQ ID N°40, SEQ ID N°41, SEQ ID N°42, SEQ ID N°66, SEQ ID N°67, SEQ ID N°68, SEQ ID N°69,
- jenen, welche durch das Teil des Gens *gag* in Einklang mit der Erfindung gebildet sind, das heißt: SEQ ID N°43, SEQ ID N°44, SEQ N°45, SEQ ID N°46, SEQ ID N°47, SEQ ID N°48, SEQ ID N°49, SEQ ID N°70, SEQ ID N°71, SEQ ID N°72, SEQ ID N°73.

7. Immunogene Zusammensetzungen, **dadurch gekennzeichnet, daß** sie ein oder mehrere Produkte der Übersetzung der Nukleotidsequenzen nach Anspruch4 und/oder mindestens eines der Peptide nach dem Anspruch 6 umfassen.

8. Antikörper gerichtet gegen eines oder mehrere der Peptide nach dem Anspruch 6.

9. In vitro Diagnoseverfahren für ein VIH-1 der Gruppe O, **dadurch gekennzeichnet, daß** es die Kontaktierung einer einem Patienten entnommenen biologischen mit Antikörpern nach dem Anspruch 8 und die Erfassung von zwischen den VIH-1 Antikörpern, welche möglicherweise in der biologischen Probe vorhanden sind, und den besagten Antikörpern gebildeten Komplexen umfaßt.

10. Verfahren zur Siebung und Typisierung von VIH1 der Gruppe O, **dadurch gekennzeichnet, daß** es die Kontaktierung eines der Nukleinsäureteile nach dem Anspruch 4 mit der Nukleinsäure des zu typisierenden Virus und die Erfassung des gebildeten Bastards umfaßt.

11. Diagnosereagenz für ein VIH-1 der Gruppe 0, **dadurch gekennzeichnet, daß** es eine Sequenz nach dem Anspruch 4 oder 6 umfaßt.

12. Diagnosereagenz, **dadurch gekennzeichnet, daß** es ausgewählt ist aus der Gruppe gebildet aus den Sequenzen ID N°22, 23, 24 et 25, welche Sequenzen geeignet sind, um als Initiator für die Verstärkung eines gp41 Teils eines VIH-1 Virus der Gruppe O zu dienen.

## Claims

1. Group O HIV-1 strains, **characterized in that** they comprise at least one of the sequences selected from the group consisting of the sequences SEQ ID No. 1 to SEQ ID No. 7 or SEQ ID No. 50 to SEQ ID No. 53, at least one of the sequences selected from the group consisting of the sequences SEQ ID No. 8 to SEQ ID No. 14 or SEQ ID No. 54 to SEQ ID No. 57 and at least one of the sequences selected from the group consisting of the sequences SEQ ID No. 15 to SEQ ID No. 21 or SEQ ID No. 58 to SEQ ID No. 61.

2. Group O HIV-1 strains according to Claim 1, **characterized in that** they are selected from the group consisting of:
- the strain which comprises the sequences SEQ ID No. 1, SEQ ID No. 8 and SEQ ID No. 15, called BCF02;
- the strain which comprises the sequences SEQ ID No. 2, SEQ ID No. 9 and SEQ ID No. 16, called BCF08;
- the strain which comprises the sequences SEQ ID No. 3, SEQ ID No. 10 and SEQ ID No. 17, called BCF03;
- the strain which comprises the sequences SEQ ID No. 4, SEQ ID No. 11 and SEQ ID No. 18, called BCF06;
- the strain which comprises the sequences SEQ ID No. 5, SEQ ID No. 12 and SEQ ID No. 19, called BCF07;
- the strain which comprises the sequences SEQ ID No. 6, SEQ ID No. 13 and SEQ ID No. 20, called BCF01;
- the strain which comprises the sequences SEQ ID No. 7, SEQ ID No. 14 and SEQ ID No. 21, called BCF11;
- the strain which comprises the sequences SEQ ID No. 50, SEQ ID No. 54, SEQ ID No. 58, called BCF09;
- the strain which comprises the sequences SEQ ID No. 51, SEQ ID No. 55, SEQ ID No. 59, called BCF12;
- the strain which comprises the sequences SEQ ID No. 52, SEQ ID No. 56, SEQ ID No. 60, called BCF13;
- the strain which comprises the sequences SEQ ID No. 53, SEQ ID No. 57, SEQ ID No. 61, called BCF14.

3. Group O HIV-1 strains according to Claim 2, deposited at the Collection Nationale de Cultures de Microorganismes held by Institut Pasteur under the numbers I-1544 (called BCF02 (ESS)), I-1543 (called BCF01 (FAN)), I-1546 (called BCF07 (MAN)), I-1547 (called BCF08 (NKO)), I-1545 (called BCF03 (POC)), on the date of 24 February 1995.

4. Nucleic acid fragment derived from a group O HIV-1, **characterized in that** its nucleotide sequence is chosen from those which are contained in one of the nucleotide sequences included in the *env* or *gag* genes and comprises:
- either one of the sequences SEQ ID No. 1, SEQ ID No. 2, SEQ ID No. 3, SEQ ID No. 4, SEQ ID No. 5, SEQ ID No. 6, SEQ ID No. 7, SEQ ID No. 50, SEQ ID No. 51, SEQ ID No. 52, SEQ ID No. 53, included in the C2V3-*env* gene fragment (hypervariable loop of gp120);
- or one of the sequences SEQ ID No. 8, SEQ ID No. 9, SEQ ID No. 10, SEQ ID No. 11, SEQ ID No. 12, SEQ ID No. 13, SEQ ID No. 14, SEQ ID No. 54, SEQ ID No. 55, SEQ ID No. 56, SEQ ID No. 57, included in the gp41 fragment of the *env* gene;
- or one of the sequences SEQ ID No. 15, SEQ ID No. 16, SEQ ID No. 17, SEQ ID No. 18, SEQ ID No. 19, SEQ ID No. 20, SEQ ID No. 21, SEQ ID No. 58, SEQ ID No. 59, SEQ ID No. 60, SEQ ID No. 61, included in the *gag* gene.

5. Process for the detection of a group O HIV-1 by hybridization or gene amplification, carried out using a biological sample (serum, circulating lymphocytes), said process being **characterized in that** it comprises:
. a step of extracting the nucleic acid to be detected, belonging to the genome of the HIV-1 type virus, which may be present in the biological sample and, where appropriate, a step of treating the nucleic acid with the aid of a reverse transcriptase, if said nucleic acid is in RNA form,
. at least one cycle comprising the steps of denaturation of the nucleic acid, annealing with at least one sequence according to Claim 4 and extension of the hybrid formed, in the presence of the appropriate reagents (polymerizing agent such as DNA polymerase and dNTP), and
. a step of detecting the possible presence of the nucleic acid belonging to the genome of a group 0 HIV-1 type virus.

6. Peptide, **characterized in that** it is expressed by an HIV-1 strain according to Claim 1 or Claim 2, or is encoded by a nucleotide sequence according to Claim 4, and **in that** it is selected from the group consisting of:
- those expressed by the C2V3-*env* gene fragment in accordance with the invention, namely: SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35, SEQ ID No. 62, SEQ ID No. 63, SEQ ID No. 64, SEQ ID No. 65,
- those expressed by the gp41 *env* gene fragment in accordance with the invention, namely: SEQ ID No. 36, SEQ ID No. 37, SEQ ID No. 38, SEQ ID No. 39, SEQ ID No. 40, SEQ ID No. 41, SEQ ID No. 42, SEQ ID No. 66, SEQ ID No. 67, SEQ ID No. 68, SEQ ID No. 69,
- those expressed by the *gag* gene fragment in accordance with the invention, namely: SEQ ID No. 43, SEQ ID No. 44, SEQ ID No. 45, SEQ ID No. 46, SEQ ID No. 47, SEQ ID No. 48, SEQ ID No. 49, SEQ ID No. 70, SEQ ID No. 71, SEQ ID No. 72, SEQ ID No. 73.

7. Immunogenic compositions, **characterized in that** they comprise one or more products of translation of the nucleotide sequences according to Claim 4 and/or at least one of the peptides according to Claim 6.

8. Antibodies directed against one or more of the peptides according to Claim 6.

9. Method of *in vitro* diagnosis of a group O HIV-1, **characterized in that** it comprises bringing a biological sample collected from a patient into contact with antibodies according to Claim 8 and detecting the immunological complexes formed between the HIV-1 antigens which may be present in the biological sample and said antibodies.

10. Process for screening and typing group O HIV-1, **characterized in that** it comprises bringing any of the nucleotide fragments according to Claim 4 into contact with the nucleic acid of the virus to be typed and detecting the hybrid formed.

11. Diagnostic reagent for a group O HIV-1, **characterized in that** it comprises a sequence according to Claim 4 or 6.

12. Diagnostic reagent, **characterized in that** it is selected from the group consisting of the sequences ID Nos. 22, 23, 24 and 25, said sequences being capable of serving as primers for the amplification of a gp41 fragment from a group O HIV-1 virus.
